# EUROPEAN PATENT APPLICATION

(11) **EP 2 092 937 A2**
(43) Date of publication of application: **26.08.2009**
(21) Application number: 09003887.8
(22) Date of filing: 02.04.2003
(51) Int. Cl.: A61K 38/16, C07K 7/08, C07K 14/415, A61K 38/10

(54) **Immunotherapeutic and immunoprophylactic reagents**

(30) Priority: 02.04.2002 AU PS148202
(62) Divisional of application: 03709428.1
(71) Applicant: Circassia Limited, Oxford, Oxfordshire OX4 4GA (GB)
(72) Inventor: O'Hehir, Robyn, Melbourne, VIC 3004 (AU); Rolland, Jennifer, Melbourne, VIC 3004 (AU)
(74) Representative: Ali, Suleman

(57) **Abstract**

The present invention relates generally to molecules such as peptides, polypeptides and proteins which interact immunologically with T lymphocytes in subjects having Rye grass pollen allergy and genetic sequences encoding same. These molecules are preferentially immunointeractive with T cells in subjects having a Rye grass pollen allergy. The molecules of the present invention are useful in the development of diagnostic, therapeutic and prophylactic agents for conditions characterised by an aberrant, inappropriate or otherwise unwanted immune response to Rye grass pollen or derivative or homologue thereof.

SEQUENCE LISTING

<110>  MONASH UNIVERSITY

<120>  Immunotherapeutic and immunoprophylactic reagents

<130>  12187260/TDO

<150>  PS1482
<151>  2002-04-02

<160>  61

<170>  PatentIn version 3.1

<210>  1
<211>  241
<212>  PRT
<213>  Lolium perenne

<400>  1

Ile Ala Lys Val Pro Pro Gly Pro Asn Ile Thr Ala Glu Tyr Gly Asp
1               5                   10                  15

Lys Trp Leu Asp Ala Lys Ser Thr Trp Tyr Gly Lys Pro Thr Gly Ala
            20                  25                  30

Gly Pro Lys Asp Asn Cys Gly Ala Cys Gly Tyr Lys Asp Val Asp Lys
        35                  40                  45

Ala Pro Phe Asn Gly Met Thr Gly Cys Gly Asn Thr Pro Ile Phe Lys
    50                  55                  60

Asp Gly Arg Gly Cys Gly Ser Cys Phe Glu Ile Lys Cys Thr Lys Pro
65                  70                  75                  80

Glu Ser Cys Ser Gly Glu Ala Val Thr Val Thr Ile Thr Asp Asp Asn
                85                  90                  95

Glu Glu Pro Ile Ala Pro Tyr His Phe Asp Leu Ser Gly His Ala Phe
            100                 105                 110

Gly Ser Met Ala Lys Lys Gly Glu Glu Gln Lys Leu Arg Ala Ser Ala
        115                 120                 125

Gly Glu Leu Glu Leu Gln Phe Arg Arg Val Lys Cys Lys Tyr Pro Asp
    130                 135                 140

Gly Thr Lys Pro Thr Phe His Val Glu Lys Ala Ser Asn Pro Asn Tyr
145                 150                 155                 160

Leu Ala Ile Leu Val Lys Tyr Val Asp Gly Asp Gly Asp Val Val Ala
                165                 170                 175

Val Asp Ile Lys Glu Lys Gly Lys Asp Lys Trp Ile Glu Leu Lys Glu
            180                 185                 190

Ser Trp Gly Ala Val Trp Arg Ile Asp Thr Pro Asp Lys Leu Thr Gly
        195                 200                 205

Pro Phe Thr Val Arg Tyr Thr Thr Glu Gly Gly Thr Lys Ser Glu Val
    210                 215                 220

Glu Asp Val Ile Pro Glu Gly Trp Lys Ala Asp Thr Ser Tyr Ser Ala
225                 230                 235                 240

Lys

<210>  2
<211>  276
<212>  PRT
<213>  Lolium perenne

<400>  2

Ala Asp Ala Gly Tyr Thr Pro Ala Ala Ala Ala Thr Pro Ala Thr Pro
1               5                   10                  15

Ala Ala Thr Pro Ala Ala Ala Gly Gly Lys Ala Thr Thr Asp Glu Gln
            20                  25                  30

Lys Leu Leu Glu Asp Val Asn Ala Gly Phe Lys Ala Ala Val Ala Ala
        35                  40                  45

Ala Ala Asn Ala Pro Pro Ala Asp Lys Phe Lys Ile Phe Glu Ala Ala
    50                  55                  60

Phe Ser Glu Ser Ser Lys Gly Leu Leu Ala Thr Ser Ala Ala Lys Ala
65                  70                  75                  80

Pro Gly Leu Ile Pro Lys Leu Asp Thr Ala Tyr Asp Val Ala Tyr Lys

85                  90                  95

Ala Ala Glu Gly Ala Thr Pro Glu Ala Lys Tyr Asp Ala Phe Val Thr
            100                 105                 110

Ala Leu Thr Glu Ala Leu Arg Val Ile Ala Gly Ala Leu Glu Val His
        115                 120                 125

Ala Val Lys Pro Ala Thr Glu Glu Val Pro Ala Ala Lys Ile Pro Thr
    130                 135                 140

Gly Glu Leu Gln Ile Val Asp Lys Ile Asp Ala Ala Phe Lys Ile Ala
145                 150                 155                 160

Ala Thr Ala Ala Asn Ala Ala Pro Thr Asn Asp Lys Phe Thr Val Phe
                165                 170                 175

Glu Ser Ala Phe Asn Lys Ala Leu Asn Glu Cys Thr Gly Gly Ala Tyr
            180                 185                 190

Glu Thr Tyr Lys Phe Ile Pro Ser Leu Glu Ala Ala Val Lys Gln Ala
        195                 200                 205

Tyr Ala Ala Thr Val Ala Ala Ala Pro Glu Val Lys Tyr Ala Val Phe
    210                 215                 220

Glu Ala Ala Leu Thr Lys Ala Ile Thr Ala Met Thr Gln Ala Gln Lys
225                 230                 235                 240

Ala Gly Lys Pro Ala Ala Ala Ala Ala Thr Gly Ala Ala Thr Val Ala
                245                 250                 255

Thr Gly Ala Ala Thr Ala Ala Ala Gly Ala Ala Thr Ala Ala Ala Gly
            260                 265                 270

Gly Tyr Lys Ala
        275

<210>  3
<211>  20
<212>  PRT
<213>  Lolium perenne

<400>  3

Ile Ala Lys Val Pro Pro Gly Pro Asn Ile Thr Ala Glu Tyr Gly Asp
1               5                   10                  15

Lys Trp Leu Asp
            20

<210>  4
<211>  20
<212>  PRT
<213>  Lolium perenne

<400>  4

Ile Thr Ala Glu Tyr Gly Asp Lys Trp Leu Asp Ala Lys Ser Thr Trp
1               5                   10                  15

Tyr Gly Lys Pro
            20

<210>  5
<211>  20
<212>  PRT
<213>  Lolium perenne

<400>  5

Leu Asp Ala Lys Ser Thr Trp Tyr Gly Lys Pro Thr Gly Ala Gly Pro
1               5                   10                  15

Lys Asp Asn Gly
            20

<210>  6
<211>  20
<212>  PRT
<213>  Lolium perenne

<400>  6

Lys Pro Thr Gly Ala Gly Pro Lys Asp Asn Gly Gly Ala Cys Gly Tyr
1               5                   10                  15

Lys Asp Val Asp
            20

<210>  7
<211>  20
<212>  PRT

<213>  Lolium perenne

<400>  7

Asn Gly Gly Ala Cys Gly Tyr Lys Asp Val Asp Lys Ala Pro Phe Asn
1               5                   10                  15

Gly Met Thr Gly
            20

<210>  8
<211>  20
<212>  PRT
<213>  Lolium perenne

<400>  8

Val Asp Lys Ala Pro Phe Asn Gly Met Thr Gly Cys Gly Asn Thr Pro
1               5                   10                  15

Ile Phe Lys Asp
            20

<210>  9
<211>  20
<212>  PRT
<213>  Lolium perenne

<400>  9

Thr Gly Cys Gly Asn Thr Pro Ile Phe Lys Asp Gly Arg Gly Cys Gly
1               5                   10                  15

Ser Cys Phe Glu
            20

<210>  10
<211>  20
<212>  PRT
<213>  Lolium perenne

<400>  10

Lys Asp Gly Arg Gly Cys Gly Ser Cys Phe Glu Ile Lys Cys Thr Lys
1               5                   10                  15

Pro Glu Ser Cys
            20

<210>  11
<211>  20
<212>  PRT
<213>  Lolium perenne

<400>  11

Phe Glu Ile Lys Cys Thr Lys Pro Glu Ser Cys Ser Gly Glu Ala Val
1               5                   10                  15

Thr Val Thr Ile
            20

<210>  12
<211>  20
<212>  PRT
<213>  Lolium perenne

<400>  12

Ser Cys Ser Gly Glu Ala Val Thr Val Thr Ile Thr Asp Asp Asn Glu
1               5                   10                  15

Glu Pro Ile Ala
            20

<210>  13
<211>  20
<212>  PRT
<213>  Lolium perenne

<400>  13

Thr Ile Thr Asp Asp Asn Glu Glu Pro Ile Ala Pro Tyr His Phe Asp
1               5                   10                  15

Leu Ser Gly His
            20

<210>  14
<211>  20
<212>  PRT
<213>  Lolium perenne

<400>  14

Ile Ala Pro Tyr His Phe Asp Leu Ser Gly His Ala Phe Gly Ser Met
1               5                   10                  15

Ala Lys Lys Gly

            20

<210>  15
<211>  20
<212>  PRT
<213>  Lolium perenne

<400>  15

Gly His Ala Phe Gly Ser Met Ala Lys Lys Gly Glu Glu Gln Asn Val
1               5                   10                  15

Arg Ser Ala Gly
            20

<210>  16
<211>  20
<212>  PRT
<213>  Lolium perenne

<400>  16

Lys Gly Glu Glu Gln Asn Val Arg Ser Ala Gly Glu Leu Glu Leu Gln
1               5                   10                  15

Phe Arg Arg Val
            20

<210>  17
<211>  20
<212>  PRT
<213>  Lolium perenne

<400>  17

Ala Gly Glu Leu Glu Leu Gln Phe Arg Arg Val Lys Cys Lys Tyr Pro
1               5                   10                  15

Asp Asp Thr Lys
            20

<210>  18
<211>  20
<212>  PRT
<213>  Lolium perenne

<400>  18

Arg Val Lys Cys Lys Tyr Pro Asp Asp Thr Lys Pro Thr Phe His Val
1               5                   10                  15

Glu Lys Gly Ser
            20


<210>  19
<211>  20
<212>  PRT
<213>  Lolium perenne

<400>  19

Thr Lys Pro Thr Phe His Val Glu Lys Gly Ser Asn Pro Asn Tyr Leu
1               5                   10                  15


Ala Ile Leu Val
            20


<210>  20
<211>  20
<212>  PRT
<213>  Lolium perenne

<400>  20

Gly Ser Asn Pro Asn Tyr Leu Ala Ile Leu Val Lys Tyr Val Asp Gly
1               5                   10                  15


Asp Gly Asp Val
            20


<210>  21
<211>  20
<212>  PRT
<213>  Lolium perenne

<400>  21

Leu Val Lys Tyr Val Asp Gly Asp Gly Asp Val Val Ala Val Asp Ile
1               5                   10                  15


Lys Glu Lys Gly
            20


<210>  22
<211>  20
<212>  PRT
<213>  Lolium perenne

<400>  22

Asp Val Val Ala Val Asp Ile Lys Glu Lys Gly Lys Asp Lys Trp Ile
1               5                   10                  15

Glu Leu Lys Glu
            20

<210>  23
<211>  20
<212>  PRT
<213>  Lolium perenne

<400>  23

Lys Gly Lys Asp Lys Trp Ile Glu Leu Lys Glu Ser Trp Gly Ala Val
1               5                   10                  15

Trp Arg Ile Asp
            20

<210>  24
<211>  20
<212>  PRT
<213>  Lolium perenne

<400>  24

Lys Glu Ser Trp Gly Ala Val Trp Arg Ile Asp Thr Pro Asp Lys Leu
1               5                   10                  15

Thr Gly Pro Phe
            20

<210>  25
<211>  20
<212>  PRT
<213>  Lolium perenne

<400>  25

Ile Asp Thr Pro Asp Lys Leu Thr Gly Pro Phe Thr Val Arg Tyr Thr
1               5                   10                  15

Thr Glu Gly Gly
            20

<210>  26
<211>  20
<212>  PRT

<213>  Lolium perenne

<400>  26

Pro Phe Thr Val Arg Tyr Thr Thr Glu Gly Gly Thr Lys Ser Glu Val
1               5                   10                  15

Glu Asp Val Ile
            20

<210>  27
<211>  20
<212>  PRT
<213>  Lolium perenne

<400>  27

Gly Gly Thr Lys Ser Glu Val Glu Asp Val Ile Pro Glu Gly Trp Lys
1               5                   10                  15

Ala Asp Thr Ser
            20

<210>  28
<211>  20
<212>  PRT
<213>  Lolium perenne

<400>  28

Ser Glu Val Glu Asp Val Ile Pro Glu Gly Trp Lys Ala Asp Thr Ser
1               5                   10                  15

Tyr Ser Ala Lys
            20

<210>  29
<211>  20
<212>  PRT
<213>  Lolium perenne

<400>  29

Ala Asp Ala Gly Tyr Thr Pro Ala Ala Ala Ala Thr Pro Ala Thr Pro
1               5                   10                  15

Ala Ala Thr Pro
            20

<210>  30
<211>  20
<212>  PRT
<213>  Lolium perenne

<400>  30

Ala Ala Thr Pro Ala Thr Pro Ala Ala Thr Pro Ala Ala Ala Gly Gly
1               5                   10                  15

Lys Ala Thr Thr
            20

<210>  31
<211>  20
<212>  PRT
<213>  Lolium perenne

<400>  31

Thr Pro Ala Ala Ala Gly Gly Lys Ala Thr Thr Asp Glu Gln Lys Leu
1               5                   10                  15

Leu Glu Asp Val
            20

<210>  32
<211>  20
<212>  PRT
<213>  Lolium perenne

<400>  32

Thr Thr Asp Glu Gln Lys Leu Leu Glu Asp Val Asn Ala Gly Phe Lys
1               5                   10                  15

Ala Ala Val Ala
            20

<210>  33
<211>  20
<212>  PRT
<213>  Lolium perenne

<400>  33

Asp Val Asn Ala Gly Phe Lys Ala Ala Val Ala Ala Ala Ala Asn Ala
1               5                   10                  15

Pro Pro Ala Asp

            20

<210>  34
<211>  20
<212>  PRT
<213>  Lolium perenne

<400>  34

Val Ala Ala Ala Ala Asn Ala Pro Pro Ala Asp Lys Phe Lys Ile Phe
1               5                   10                  15

Glu Ala Ala Phe
            20

<210>  35
<211>  20
<212>  PRT
<213>  Lolium perenne

<400>  35

Ala Asp Lys Phe Lys Ile Phe Glu Ala Ala Phe Ser Glu Ser Ser Lys
1               5                   10                  15

Gly Leu Leu Ala
            20

<210>  36
<211>  20
<212>  PRT
<213>  Lolium perenne

<400>  36

Ala Phe Ser Glu Ser Ser Lys Gly Leu Leu Ala Thr Ser Ala Ala Lys
1               5                   10                  15

Ala Pro Gly Leu
            20

<210>  37
<211>  20
<212>  PRT
<213>  Lolium perenne

<400>  37

Leu Ala Thr Ser Ala Ala Lys Ala Pro Gly Leu Ile Pro Lys Leu Asp
1               5                   10                  15

Thr Ala Tyr Asp
            20

<210>  38
<211>  20
<212>  PRT
<213>  Lolium perenne

<400>  38

Gly Leu Ile Pro Lys Leu Asp Thr Ala Tyr Asp Val Ala Tyr Lys Ala
1               5                   10                  15

Ala Glu Gly Ala
            20

<210>  39
<211>  20
<212>  PRT
<213>  Lolium perenne

<400>  39

Tyr Asp Val Ala Tyr Lys Ala Ala Glu Gly Ala Thr Pro Glu Ala Lys
1               5                   10                  15

Tyr Asp Ala Phe
            20

<210>  40
<211>  20
<212>  PRT
<213>  Lolium perenne

<400>  40

Gly Ala Thr Pro Glu Ala Lys Tyr Asp Ala Phe Val Thr Ala Leu Thr
1               5                   10                  15

Glu Ala Leu Arg
            20

<210>  41
<211>  20
<212>  PRT
<213>  Lolium perenne

<400>  41

Ala Phe Val Thr Ala Leu Thr Glu Ala Leu Arg Val Ile Ala Gly Ala
1               5                   10                  15

Leu Glu Val His
            20

<210>  42
<211>  20
<212>  PRT
<213>  Lolium perenne

<400>  42

Leu Arg Val Ile Ala Gly Ala Leu Glu Val His Ala Val Lys Pro Ala
1               5                   10                  15

Thr Glu Glu Val
            20

<210>  43
<211>  20
<212>  PRT
<213>  Lolium perenne

<400>  43

Val His Ala Val Lys Pro Ala Thr Glu Glu Val Pro Ala Ala Lys Ile
1               5                   10                  15

Pro Thr Gly Glu
            20

<210>  44
<211>  20
<212>  PRT
<213>  Lolium perenne

<400>  44

Glu Val Pro Ala Ala Lys Ile Pro Thr Gly Glu Leu Gln Ile Val Asp
1               5                   10                  15

Lys Ile Asp Ala
            20

<210>  45
<211>  20
<212>  PRT

<213>  Lolium perenne

<400>  45

Gly Glu Leu Gln Ile Val Asp Lys Ile Asp Ala Ala Phe Lys Ile Ala
1               5                   10                  15

Ala Thr Ala Ala
            20

<210>  46
<211>  20
<212>  PRT
<213>  Lolium perenne

<400>  46

Asp Ala Ala Phe Lys Ile Ala Ala Thr Ala Ala Asn Ala Ala Pro Thr
1               5                   10                  15

Asn Asp Lys Phe
            20

<210>  47
<211>  20
<212>  PRT
<213>  Lolium perenne

<400>  47

Ala Ala Asn Ala Ala Pro Thr Asn Asp Lys Phe Thr Val Phe Glu Ser
1               5                   10                  15

Ala Phe Asn Lys
            20

<210>  48
<211>  20
<212>  PRT
<213>  Lolium perenne

<400>  48

Lys Phe Thr Val Phe Glu Ser Ala Phe Asn Lys Ala Leu Asn Glu Cys
1               5                   10                  15

Thr Gly Gly Ala
            20

<210>  49
<211>  20
<212>  PRT
<213>  Lolium perenne

<400>  49

Asn Lys Ala Leu Asn Glu Cys Thr Gly Gly Ala Tyr Glu Thr Tyr Lys
1               5                   10                  15

Phe Ile Pro Ser
            20

<210>  50
<211>  20
<212>  PRT
<213>  Lolium perenne

<400>  50

Gly Ala Tyr Glu Thr Tyr Lys Phe Ile Pro Ser Leu Glu Ala Ala Val
1               5                   10                  15

Lys Gln Ala Tyr
            20

<210>  51
<211>  20
<212>  PRT
<213>  Lolium perenne

<400>  51

Pro Ser Leu Glu Ala Ala Val Lys Gln Ala Tyr Ala Ala Thr Val Ala
1               5                   10                  15

Ala Ala Pro Glu
            20

<210>  52
<211>  20
<212>  PRT
<213>  Lolium perenne

<400>  52

Ala Tyr Ala Ala Thr Val Ala Ala Ala Pro Glu Val Lys Tyr Ala Val
1               5                   10                  15

Phe Glu Ala Ala

            20

<210>  53
<211>  20
<212>  PRT
<213>  Lolium perenne

<400>  53

Pro Glu Val Lys Tyr Ala Val Phe Glu Ala Ala Leu Thr Lys Ala Ile
1               5                   10                  15

Thr Ala Met Thr
            20

<210>  54
<211>  20
<212>  PRT
<213>  Lolium perenne

<400>  54

Ala Ala Leu Thr Lys Ala Ile Thr Ala Met Thr Gln Ala Gln Lys Ala
1               5                   10                  15

Gly Lys Pro Ala
            20

<210>  55
<211>  20
<212>  PRT
<213>  Lolium perenne

<400>  55

Met Thr Gln Ala Gln Lys Ala Gly Lys Pro Ala Ala Ala Ala Ala Thr
1               5                   10                  15

Gly Ala Ala Thr
            20

<210>  56
<211>  20
<212>  PRT
<213>  Lolium perenne

<400>  56

Pro Ala Ala Ala Ala Ala Thr Gly Ala Ala Thr Val Ala Thr Gly Ala
1               5                   10                  15

Ala Thr Ala Ala
            20


<210>  57
<211>  20
<212>  PRT
<213>  Lolium perenne

<400>  57

Ala Thr Val Ala Thr Gly Ala Ala Thr Ala Ala Ala Gly Ala Ala Thr
1               5                   10                  15


Ala Ala Ala Gly
            20


<210>  58
<211>  20
<212>  PRT
<213>  Lolium perenne

<400>  58

Thr Gly Ala Ala Thr Ala Ala Ala Gly Ala Ala Thr Ala Ala Ala Gly
1               5                   10                  15


Gly Tyr Lys Ala
            20


<210>  59
<211>  276
<212>  PRT
<213>  Lolium perenne

<400>  59

Ala Asp Ala Gly Tyr Thr Pro Ala Ala Ala Ala Thr Pro Ala Thr Pro
1               5                   10                  15


Ala Ala Thr Pro Ala Ala Ala Gly Gly Lys Ala Thr Thr Asp Glu Gln
            20                  25                  30


Lys Leu Leu Glu Asp Val Asn Ala Gly Phe Lys Ala Ala Val Ala Ala
        35                  40                  45


Ala Ala Asn Ala Pro Pro Ala Asp Lys Phe Lys Ile Phe Glu Ala Ala
    50                  55                  60

Phe Ser Glu Ser Ser Lys Gly Leu Leu Ala Thr Ser Ala Ala Lys Ala
65                  70                  75                  80

Pro Gly Leu Ile Pro Lys Leu Asp Thr Ala Tyr Asp Val Ala Tyr Lys
                85                  90                  95

Ala Ala Glu Gly Ala Thr Pro Glu Ala Lys Tyr Asp Ala Phe Val Thr
            100                 105                 110

Ala Leu Thr Glu Ala Leu Arg Val Ile Ala Gly Ala Leu Glu Val His
        115                 120                 125

Ala Val Lys Pro Ala Thr Glu Glu Val Pro Ala Ala Lys Ile Pro Thr
    130                 135                 140

Gly Glu Leu Gln Ile Val Asp Lys Ile Asp Ala Ala Phe Lys Ile Ala
145                 150                 155                 160

Ala Thr Ala Ala Asn Ala Ala Pro Thr Asn Asp Lys Phe Thr Val Phe
                165                 170                 175

Glu Ser Ala Phe Asn Lys Ala Leu Asn Glu Cys Thr Gly Gly Ala Tyr
            180                 185                 190

Glu Thr Tyr Lys Phe Ile Pro Ser Leu Glu Ala Ala Val Lys Gln Ala
        195                 200                 205

Tyr Ala Ala Thr Val Ala Ala Ala Pro Glu Val Lys Tyr Ala Val Phe
    210                 215                 220

Glu Ala Ala Leu Thr Lys Ala Ile Thr Ala Met Thr Gln Ala Gln Lys
225                 230                 235                 240

Ala Gly Lys Pro Ala Ala Ala Ala Ala Thr Gly Ala Ala Thr Val Ala
                245                 250                 255

Thr Gly Ala Ala Thr Ala Ala Ala Gly Ala Ala Thr Ala Ala Ala Gly
            260                 265                 270

Gly Tyr Lys Ala
        275

<210>  60
<211>  276
<212>  PRT
<213>  Lolium perenne

<400>  60

Ala Asp Ala Gly Tyr Thr Pro Ala Ala Ala Ala Thr Pro Ala Thr Pro
1               5                   10                  15

Ala Ala Thr Pro Ala Ala Gly Gly Gly Lys Ala Thr Thr Asp Glu Gln
            20                  25                  30

Lys Leu Leu Glu Asp Val Asn Ala Gly Phe Lys Ala Ala Val Ala Ala
        35                  40                  45

Asp Ala Asn Ala Pro Pro Ala Asp Lys Phe Lys Ile Phe Glu Ala Ala
    50                  55                  60

Phe Ser Glu Ser Cys Lys Gly Leu Leu Ala Thr Ser Asp Ala Lys Ala
65                  70                  75                  80

Pro Gly Leu Ile Leu Lys Leu Asp Thr Asp Tyr Asp Val Ala Tyr Lys
                85                  90                  95

Ala Gly Glu Gly Ala Thr Pro Glu Ala Lys Tyr Asp Ala Phe Val Thr
            100                 105                 110

Ala Leu Thr Glu Ala Leu Arg Val Ile Ala Gly Ala Leu Glu Val His
        115                 120                 125

Ala Val Lys Pro Ala Thr Glu Glu Val Pro Ala Ala Lys Ile Pro Thr
    130                 135                 140

Gly Glu Leu Gln Ile Val Asp Lys Ile Asp Ala Ala Phe Lys Ile Ala
145                 150                 155                 160

Ala Thr Ala Ala Asn Ala Ala Pro Thr Asn Asp Lys Phe Thr Val Phe
                165                 170                 175

Glu Ser Ala Phe Asn Lys Ala Leu Lys Glu Cys Thr Gly Gly Ala Tyr
            180                 185                 190

Glu Thr Tyr Lys Phe Ile Pro Ser Leu Glu Ala Ala Val Lys Gln Ala

195                 200                 205

Tyr Ala Thr Thr Val Ala Ala Ala Pro Glu Val Lys Tyr Ala Val Phe
    210                 215                 220

Glu Ala Ala Leu Thr Lys Ala Ile Thr Ala Met Ser Gln Ala Gln Lys
225                 230                 235                 240

Val Ala Lys Pro Ala Ala Ala Ala Ala Thr Gly Ala Ala Thr Val Ala
                245                 250                 255

Thr Gly Ala Ala Thr Ala Ala Ala Gly Gly Ala Thr Ala Ala Ala Gly
            260                 265                 270

Gly Tyr Lys Ala
        275

<210>  61
<211>  314
<212>  PRT
<213>  Lolium perenne

<400>  61

Ala Asp Ala Gly Tyr Ala Pro Ala Thr Pro Ala Thr Pro Ala Ala Pro
1               5                   10                  15

Ala Thr Ala Ala Thr Pro Ala Thr Pro Ala Thr Pro Ala Thr Pro Ala
            20                  25                  30

Ala Val Pro Ser Gly Lys Ala Thr Thr Glu Glu Gln Lys Leu Ile Glu
        35                  40                  45

Lys Ile Asn Ala Gly Phe Lys Ala Ala Val Ala Ala Ala Ala Val Val
    50                  55                  60

Pro Pro Ala Asp Lys Tyr Lys Thr Phe Val Glu Thr Phe Gly Thr Ala
65                  70                  75                  80

Thr Asn Lys Ala Phe Val Glu Gly Leu Ala Ser Gly Tyr Ala Asp Gln
                85                  90                  95

Ser Lys Asn Gln Leu Thr Ser Lys Leu Asp Ala Ala Leu Lys Leu Ala
            100                 105                 110

Tyr Glu Ala Ala Gln Gly Ala Thr Pro Glu Ala Lys Tyr Asp Ala Tyr
        115                 120                 125

Val Ala Thr Leu Thr Glu Ala Leu Arg Val Ile Ala Gly Thr Leu Glu
    130                 135                 140

Val His Ala Val Lys Pro Ala Ala Glu Glu Val Lys Val Gly Ala Ile
145                 150                 155                 160

Pro Ala Ala Glu Val Gln Leu Ile Asp Lys Val Asp Ala Ala Tyr Arg
                165                 170                 175

Thr Ala Ala Thr Ala Ala Asn Ala Ala Pro Ala Asn Asp Lys Phe Thr
            180                 185                 190

Val Phe Glu Asn Thr Phe Asn Asn Ala Ile Lys Val Ser Leu Gly Ala
        195                 200                 205

Ala Tyr Asp Ser Tyr Lys Phe Ile Pro Thr Leu Val Ala Ala Val Lys
    210                 215                 220

Gln Ala Tyr Ala Ala Lys Gln Ala Thr Ala Pro Glu Val Lys Tyr Thr
225                 230                 235                 240

Val Ser Glu Thr Ala Leu Lys Lys Ala Val Thr Ala Met Ser Glu Ala
                245                 250                 255

Glu Lys Glu Ala Thr Pro Ala Ala Ala Ala Thr Ala Thr Pro Thr Pro
            260                 265                 270

Ala Ala Ala Thr Ala Thr Ala Thr Pro Ala Ala Ala Tyr Ala Thr Ala
        275                 280                 285

Thr Pro Ala Ala Ala Thr Ala Thr Ala Thr Pro Ala Ala Ala Thr Ala
    290                 295                 300

Thr Pro Ala Ala Ala Gly Gly Tyr Lys Val
305                 310

## Description

### FIELD OF THE INVENTION

The present invention relates generally to molecules such as peptides, polypeptides and proteins which interact immunologically with T lymphocytes in subjects having Rye grass pollen allergy and genetic sequences encoding same. These molecules are preferentially immunointeractive with T cells in subjects having a Rye grass pollen allergy. The molecules of the present invention are useful in the development of diagnostic, therapeutic and prophylactic agents for conditions characterised by an aberrant, inappropriate or otherwise unwanted immune response to Rye grass pollen or derivative or homologue thereof.

### BACKGROUND OF THE INVENTION

Bibliographic details of the publications referred to by author in this specification are collected alphabetically at the end of the description.

The reference to any prior art in this specification is not, and should not be taken as, an acknowledgment or any form of suggestion that that prior art forms part of the common general knowledge in Australia.

Type I allergic diseases such as seasonal allergic rhinitis (hayfever), conjunctivitis, allergic asthma and allergic dermatitis represent a major health problem in industrialised countries (Wuthrich, 1989). It is currently estimated that 15-20% of the population in developed countries are afflicted with some form of allergy (Miyamoto, 1992). Therefore, the diagnosis and therapy of these diseases have become focal points of interest for scientific investigation.

At present, one form of therapeutic intervention of allergic diseases (such as rhinitis and allergic asthma) involves injection of the allergen assumed to be responsible for the allergic response. This is referred to as hyposensitisation treatment. The development of recombinant techniques has provided the means to produce high levels of purified allergens for such purposes. However, the high level of purity of recombinant allergen preparations results in a high anaphylactogenic index even at very low doses.
Accordingly, extreme care is required when they are administered to patients. There is a need, therefore, to develop new agents and means of treating patients such as to reduce the risk of anaphylactic shock.

The major outdoor cause of seasonal hay-fever and allergic asthma is airborne grass pollen (Smart *et al.* 1983). Pollen calenders show that grass pollen is most abundant in spring and early summer when grasses flower and this is when allergic asthma peaks in incidence. The most important sources of grass pollen are common agricultural pasture grasses which have been widely introduced throughout the world. In cool temperate regions, grasses such as Rye-grass, Kentucky bluegrass and Timothy (all belonging to the subfamily Pooideae) are of clinical significance, whereas in warm temperate and subtropical environments pollen of Bermuda grass (subfamily Chloridoideae) becomes the most important source of allergens. The most comprehensive studies have been made on proteins from Rye grass pollen and to a lesser extent Kentucky bluegrass and Timothy.

Individuals sensitive to allergens from one grass are often sensitive to those of a number of other grass genera. This is particularly true for pollen of grasses within the subfamily Pooideae (Smith *et al.,* 1994), where immunological cross-reactivity has been demonstrated in inhibition experiments using an IgE-binding assay, the radioallergosorbent test (RAST). In these experiments, pollen extracts from one grass were able to inhibit binding of IgE to extracts from other grasses.

Allergen-specific CD4⁺ T cells producing Th2-type cytokines play a major role in the elicitation and progression of allergic diseases (Romagnani, 1997). Upon appropriate presentation of allergen peptide these T cells produce a cocktail of cytokines, most importantly IL-4 which drives IgE class switching by allergen-stimulated B cells and IL-5 which plays an important role in the maturation and activation of eosinophils. Allergen immunotherapy has been successfully used to specifically treat allergic disease (Bousquet *et al.,* 1998), yet the precise mechanisms involved are as yet not fully understood. Moreover, efficacy for treatment of common allergies such as those induced by grass pollens and house dust mite is only 60-80% and there is a risk of IgE-mediated side effects from injection of the current crude extracts. Several studies have shown that clinically effective immunotherapy induces an alteration in allergen-specific T cell cytokine phenotype from a predominant Th2/Th0-type to a more Th1/Th0-type (reviewed in Rolland *et al.,* 1998). Since the nature of the T cell response is pivotal in determining whether clinical tolerance or allergy follows allergen challenge, T cells are obvious targets for new immunotherapy strategies.

Based on a knowledge of dominant T cell epitopes of allergens, short peptides or recombinant hypoallergenic forms of allergens are exciting effective alternatives to current immunotherapy allergen extracts. Since these new preparations cannot cross-link mast cell-bound IgE, they should also be associated with improved safety of immunotherapy treatment. Peptide immunotherapy has already been successfully clinically trialed for the major cat allergen Fel d 1 (Pene *et al.,* 1998; Alexander *et al.,* 2001; Oldfield, 2001) and the major bee venom allergen phospholipase A2 (PLA₂)(Muller *et al.,* 1998). In addition, strategies for the generation of hypoallergenic recombinant allergens by site directed mutagenesis have been developed for birch pollen, Timothy grass pollen and house dust mite allergens (Vrtala *et al.,* 1999; Schramm *et al.,* 1999; Smith *et al.,* 1998).

Rye grass pollen (RGP) is an important aeroallergen source in cool temperate climates during the grass flowering season. A soluble extract of RGP contains at least 17 allergenic proteins as identified by immunoblotting with patient serum IgE, ranging in size from 11-89 kDa (Ford *et al.,* 1986; Stewart *et al.,* 1988). Lol p 1 and Lol p 5 are the major allergens of RGP, which together are recognised by almost all sera from RGP allergic donors. Limited mapping of T cell epitopes of Lol p 1 and Lol p 5 using PBMC, T cell lines (TCL) or T cell clones (TCC) has been reported previously (Perez *et al.,* 1990; Spiegelberg *et al.,* 1994; Bungy Poor Fard *et al.,* 1993; Bungy *et al.,* 1994; Blaher *et al.,* 1996; de Lalla *et al.,* 1999). However, these results are equivocal due to the analysis methods utilised and the small donor series.

In work leading up to the present invention, the inventors have identified the human T cell epitopes of the Rye grass pollen allergens Lol p 1 and Lol p 5. Using short-term RGP specific TCL generated from peripheral blood mononuclear cells of a large panel of RGP allergic individuals, the importance of Lol p 1 and Lol p 5 as major T cell antigens of RGP was established and several dominant T cell reactive regions were identified in both molecules. The identification of Rye grass pollen T cell epitopes now facilitates the development of molecules and methodology for the diagnosis and treatment of conditions characterised by the aberrant, inappropriate or otherwise unwanted immune response to Rye grass pollen or derivative or homologue thereof.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The subject specification contains amino acid sequence information prepared using the programme PatentIn Version 3.1, presented herein after the bibliography. Each amino acid sequence is identified in the sequence listing by the numeric indicator <201> followed by the sequence identifier (eg. <210>1, <210>2, etc). The length, type of sequence (DNA, protein (PRT), etc) and source organism for each amino acid sequence are indicated by information provided in the numeric indicator fields <211>, <212> and <213>, respectively. Amino acid sequences referred to in the specification are defined by the information provided in numeric indicator field <400> followed by the sequence identifier (eg. <400>1, <400>2, etc). That is SEQ ID NO: as detailed in the specification correlates to the sequence indicated as <400>1 in the sequence listing.

Accordingly, one aspect of the present invention provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is any amino acid sequence derived from or homologous to Lol p 1;
and wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

In another aspect of the present invention provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is any amino acid sequence derived from or homologous to Lol p 5;
and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide.

The present invention therefore more particularly provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 1-240 inclusive of Lol p 1;
and wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

Still more particularly the present invention provides an isolated peptide of the formula:

X₁X₂X₃

wherein
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 19-47, 73-92, 100-128, 127-146, 154-173 or 181-209 inclusive of Lol p 1;
and wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

Still more particularly, X₂ is any amino acid sequence of from 5 to 100 residues derived from, homologous to or contiguous with amino acids 19-38, 28-47, 73-92,100-119, 109-128,127-146, 154-173, 181-200, 190-209 inclusive of Lol p 1.

Yet more particularly, X₂ is any amino acid sequence of from 5 to 100 residues derived from homologous to or contiguous with amino acids 19-38, 109-128, 154-173 and/or 190-209 inclusive of Lol p 1.

Most particularly, X₂ is any amino acid sequence of from 5 to 100 residues derived from, homologous to or contiguous with amino acids 19-38 inclusive of Lol p 1.

Still more particularly, X₂ is any amino acid sequence of from 5 to 100 residues derived from, homologous to or contiguous with amino acids 109-128 and/or 154-173 inclusive of Lol p 1.

Still more particularly, X₂ is any amino acid sequence of from 5 to 100 residues derived from, homologous to or contiguous with amino acids 190-209 inclusive of Lol p 1.

The present invention therefore more particularly provides an isolated peptide of the formula:

X₁X₂X₃

wherein:
X₁ and X₃ may be the same or different and each is an amino acid sequence comprising from 0 to 40 naturally or non-naturally occurring amino acid residues:
X₂ is an amino acid sequence of from 5 to 100 residues derived from, homologous to or contiguous with amino acids 1-276 inclusive of Lol p 5;
and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide provided that X₂ is not the amino acid sequence 100-119 or 190-209.

Still more particularly the present invention provides an isolated peptide of the formula:

X₁X₂X₃

wherein
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 37-83, 118-137, 145-173, 172-191 or 190-245 inclusive of Lol p 5.
and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide provided that X₂ is not the amino acid sequence 100-119 or 190-209.

Still more particularly, X₂ is any amino acid sequence of from 5 to 100 residues derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.

Yet more particularly, X₂ is any amino acid sequence of from 5 to 100 residues derived from homologous to or contiguous with amino acids 37-56, 145-164, 154-173, 217-236 and/or 226-245 inclusive of Lol p 5.

Most particularly, X₂ is any amino acid sequence of from 5 to 100 residues derived from, homologous to or contiguous with amino acids 37-56 inclusive of Lol p 5.

Still more particularly, X₂ is any amino acid sequence of from 5 to 100 residues derived from, homologous to or contiguous with amino acids 145-1664 and/or 154-173 inclusive of Lol p 5.

Still more particularly, X₂ is any amino acid sequence of from 5 to 100 residues derived from, homologous to or contiguous with amino acids 217-236 and/or 226-245 inclusive of Lol p 5.

In a particularly preferred embodiment, where the subject Rye grass pollen allergen is Lol p 1, X₂ comprises a sequence of at least 5 amino acids derived from one or more of the following amino acid sequences:
LDAKSTWYGKPTGAGPKDNG (SEQ ID NO: 5)
KPTGAGPKDNGGACGYKDVD (SEQ ID NO: 6)
FEIKCTKPESCSGEAVTVTI (SEQ ID NO: 11)
IAPYHFDLSGHAFGSMAKKG (SEQ ID NO: 14)
GHAFGSMAKKGEEQNVRSAG (SEQ ID NO: 15)
AGELELQFRRVKCKYPDDTK (SEQ ID NO: 17)
GSNPNYLAILVKYVDGDGDV (SEQ ID NO: 20)
KGKDKWIELKESWGAVWRID (SEQ ID NO: 23)
KESWGAVWRIDTPDKLTGPF (SEQ ID NO: 25)

More preferably, X₂ comprises a sequence of at least 5 amino acids derived from one or more of SEQ ID NO: 5, SEQ ID NO: 15, SEQ ID NO: 20 and/or SEQ ID NO: 24.

In another preferred embodiment, where the subject Rye grass pollen allergen is Lol p 5, X₂ comprises a sequence of at least 5 amino acids derived from one or more of the following amino acid sequences:
DVNAGFKAAVAAAANAPPAD (SEQ ID NO: 33)
VAAAANAPPADKFKIFEAAF (SEQ ID NO: 34)
ADKFKIFEAAFSESSKGLLA (SEQ ID NO: 35)
AFSESSKGLLATSAAKAPGL (SEQ ID NO: 36)
LRVIAGALEVHAVKPATEEV (SEQ ID NO: 42)
GELQIVDKmAAFKLAATAA (SEQ ID NO: 45)
DAAFKIAATAANAAPTNDKF (SEQ ID NO: 46)
KFTVFESAFNKALNECTGGA (SEQ ID NO: 48)
PSLEAAVKQAYAATVAAAPE (SEQ ID NO: 51)
AYAATVAAAPEVKYAVFEAA (SEQ ID NO: 52)
PEVKYAVFEAALTKAITAMT (SEQ ID NO: 53)
AALTKAITAMTQAQKAGKPA (SEQ ID NO: 54)

More preferably, X₂ comprises a sequence of at least 5 amino acids derived from one or more of SEQ ID NO: 33, SEQ ID NO: 45, SEQ ID NO: 46, SEQ ID NO: 53 or SEQ ID NO: 54.

Another aspect of the present invention provides an isolated peptide comprising any amino acid sequence derived from or homologous to Lol p 1 or Lol p 5 wherein said peptide molecule is capable of interacting with T cells and modifying T cell function when incubated with cells from subjects having a condition characterised by an aberrant, unwanted or otherwise inappropriate immune response to Lol p 1 or Lol p 5, respectively, or a functional derivative, homologue, analogue or mutant of said peptide.

More particularly, the present invention provides an isolated peptide comprising an amino acid sequence of from 5-100 residues derived from, homologous to or contiguous with amino acids 1-240 inclusive or derivatives thereof of Lol p 1 wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

In one preferred embodiment said amino acid sequence is derived from, homologous to or contiguous with amino acids 19-47, 73-92, 100-128,127-146, 154-173 or 181-209, inclusive of Lol p 1.

In another preferred embodiment said amino acid sequence is derived from, homologous to or contiguous with amino acids 19-38, 28-47, 73-92, 100-119, 109-128, 127-146, 154-173, 181-200 and/or 190-209 inclusive of Lol p 1.

In yet another preferred embodiment said amino acid sequence is derived from, homologous to or contiguous with amino acids 19-38, 109-128, 154-173 and/or 190-209 inclusive of Lol p 1.

In another aspect, where the subject Rye grass pollen allergen is Lol p 1, said amino acid sequence comprises a sequence of at least 5 amino acids derived from one or more of the following amino acid sequences:
LDAKSTWYGKPTGAGPKDNG (SEQ ID NO: 5)
KPTGAGPKDNGGACGYKDVD (SEQ ID NO: 6)
FEIKCTKPESCSGEAVTVTI (SEQ ID NO: 11)
IAPYHFDLSGHAFGSMAKKG (SEQ ID NO: 14)
GHAFGSMAKKGEEQNVRSAG (SEQ ID NO: 15)
AGELELQFRRVKCKYPDDTK (SEQ ID NO: 17)
GSNPNYLAILVKYVDGDGDV (SEQ ID NO: 20)
KGKDKWIELKESWGAVWRID (SEQ ID NO: 23)
KESWGAVWRIDTPDKLTGPF (SEQ ID NO: 24)

According to this aspect, said amino acid sequence preferably comprises a sequence of at least 5 amino acids derived from one or more of SEQ ID NO: 5, SEQ ID NO: 15, SEQ ID NO: 20 and/or SEQ ID NO: 24.

In yet another embodiment, the present invention provides an isolated peptide comprising an amino acid sequence of from 5-100 residues derived from, homologous to or contiguous with amino acids 1-276 inclusive or derivatives thereof of Lol p 5 wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide provided that said amino acid sequence is not the amino acid sequence 100-119 or 190-209.

In one preferred embodiment said amino acid sequence is derived from, homologous to or contiguous with amino acids 37-83, 118-137, 145-173, 172-191 and/or 190-245 inclusive of Lol p 5 provided that said amino acid sequence is not the amino acid sequence 100-119 or 190-209.

In another preferred embodiment said amino acid sequence is derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.

In yet another preferred embodiment said amino acid sequence is derived from, homologous to or contiguous with amino acids 37-56, 145-164, 154-173, 217-236 and/or 226-245.

In another aspect, where the subject Rye grass pollen allergen is Lol p 5, said amino acid sequence comprises a sequence of at least 5 amino acids derived from one or more of the following amino acid sequences:
DVNAGFKAAVAAAANAPPAD (SEQ ID NO: 33)
VAAAANAPPADKFKIFEAAF (SEQ ID NO: 34)
ADKFKIFEAAFSESSKGLLA (SEQ ID NO: 35)
AFSESSKGLLATSAAKAPGL (SEQ ID NO: 36)
LRVIAGALEVHAVKPATEEV (SEQ ID NO: 42)
GELQIVDKIDAAFKIAATAA (SEQ ID NO: 45)
DAAFKIAATAANAAPTNDKF (SEQ ID NO: 46)
KFTVFESAFNKALNECTGGA (SEQ ID NO: 48)
PSLEAAVKQAYAATVAAAPE (SEQ ID NO: 51)
AYAATVAAAPEVKYAVFEAA (SEQ ID NO: 52)
PEVKYAVFEAALTKAITAMT (SEQ ID NO: 53)
AALTKAITAMTQAQKAGKPA (SEQ ID NO: 54)

According to this aspect, said amino acid sequence preferably comprises a sequence of at least 5 amino acids derived from one or more of SEQ ID NO: 33, SEQ ID NO: 45, SEQ ID NO: 46, SEQ ID NO: 53 or SEQ ID NO: 54.

In another aspect, the present invention provides a nucleic acid molecule comprising a sequence of nucleotides encoding or complementary to a sequence encoding the peptides as hereinbefore defined or a functional derivative, homologue, mutant or analogue thereof.

Accordingly, in another aspect the present invention provides a method for the treatment and/or prophylaxis of a condition in a subject, which condition is characterised by the aberrant, unwanted or otherwise inappropriate immune response to Lol p 1 and/or Lol p 5 , said method comprising administering to said subject an effective amount of a peptide as hereinbefore defined for a time and under conditions sufficient to remove or reduce the presence or function in said subject of T cells directed to said Lol p 1 and/or Lol p 5 .

Another aspect of the present invention contemplates the use of an agent as hereinbefore defined in the manufacture of a medicament for the treatment of a condition in a mammal, which condition is characterised by an aberrant, unwanted or otherwise inappropriate immune response to Lol p 1 and/or Lol p 5.

In yet another further aspect, the present invention contemplates a pharmaceutical composition comprising an agent as hereinbefore defined and one or more pharmaceutically acceptable carriers and/or diluents.

Yet another aspect of the present invention relates to agents, as hereinbefore defined, when used in the method of the present invention.

Accordingly, yet another aspect of the present invention is directed to a method of diagnosing or monitoring a condition in a mammal, which condition is characterised by an aberrant, unwanted or inappropriate response to Lol p 1 and/or Lol p 5 , said method comprising screening for Lol p 1 and/or Lol p 5 reactive T cells utilising the peptides hereinbefore defined.

In another embodiment the present invention provides diagnostic kits for use in the diagnostic methodology hereinbefore defined.

Single and three letter abbreviations used throughout the specification are defined in Table 1.

**TABLE 1**

| **Single and three letter amino acid abbreviations** | | |
|---|---|---|
| Amino Acid | Three-letter Abbreviation | One-letter Symbol |
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

### BRIEF DESCRIPTION OF THE DRAWINGS

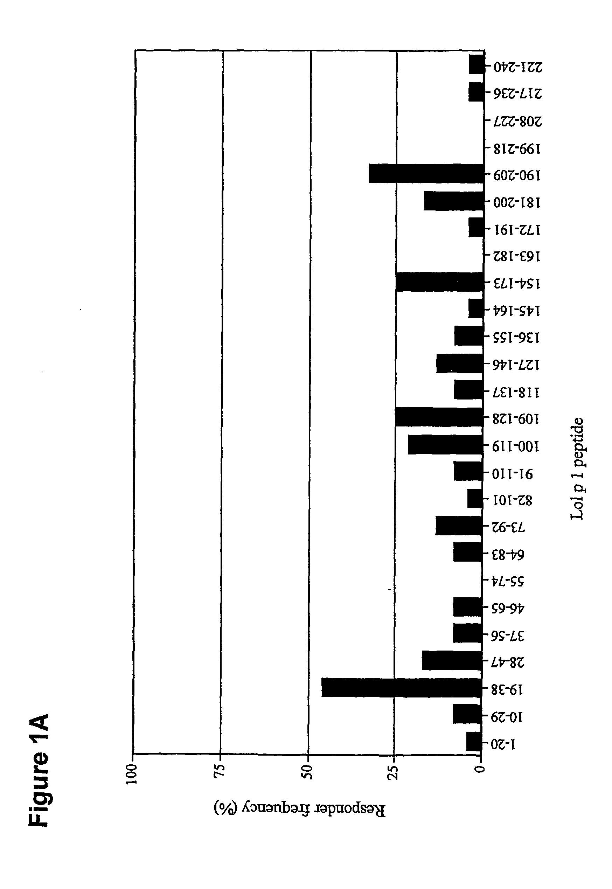

**Figure 1A** is a graphical representation of the responder frequency of TCL from a panel of 34 atopic donors to individual Lol p 1 peptides. A stimulation index of ≥ 2.5 was considered positive and the number of donor TCL responding to each peptide is shown as a percentage.

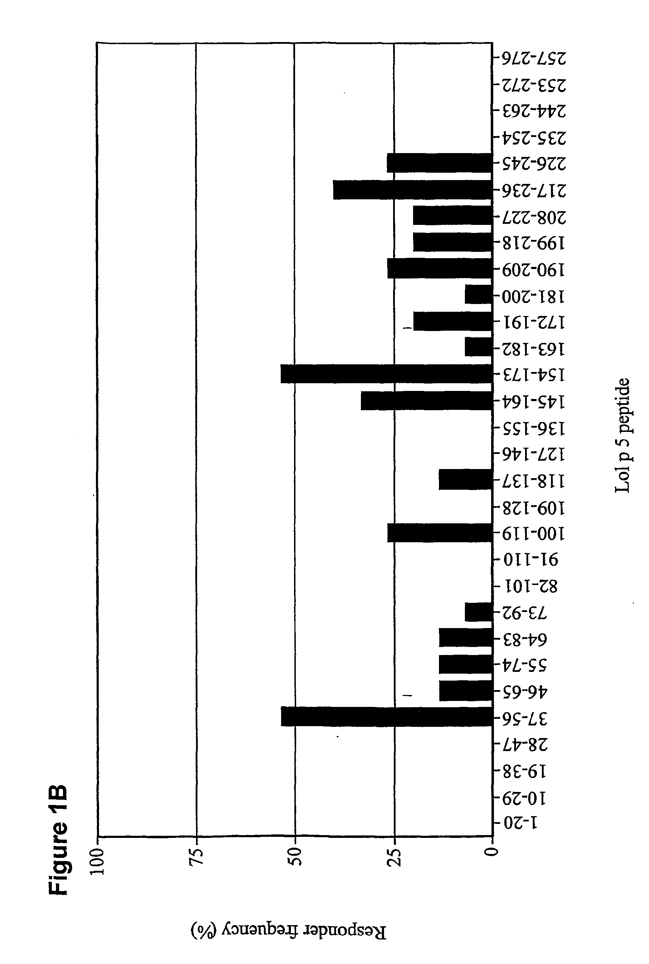

**Figure 1B** is a graphical representation of the responder frequency of TCL from a panel of 21 atopic donors to individual Lol p 5 peptides. A stimulation index of ≥ 2.5 was considered positive and the number of donor TCL responding to each peptide is shown as a percentage.

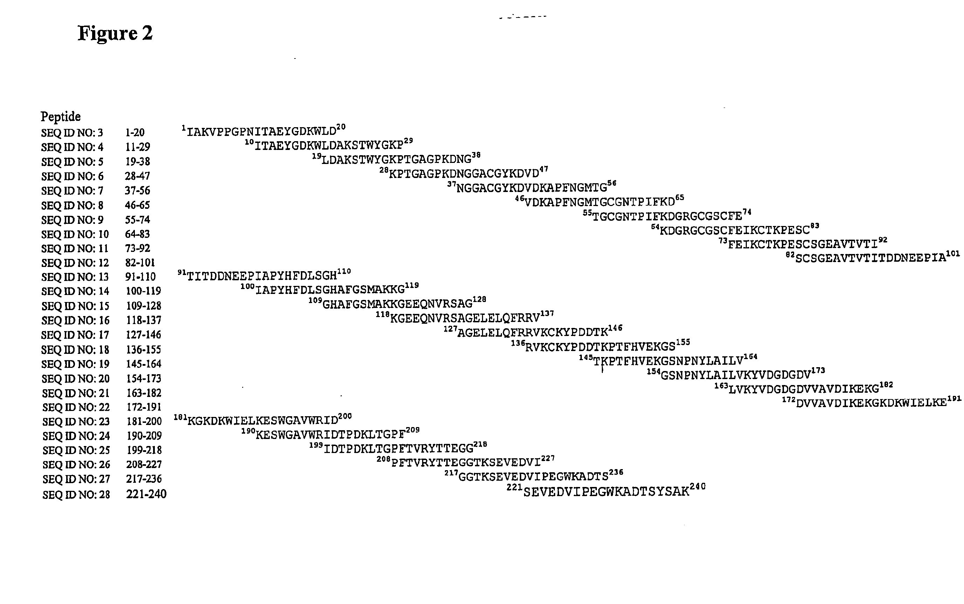

**Figure 2** is a schematic representation of the Lol p 1 20-mer peptide series. The sequence of Lol p 1 is provided in SEQ ID NO:1. The sequence of each Lol p 1 peptide is shown with the amino acid sequence number designating each peptide given to the left. The overlapping regions of adjacent peptides (spanning 11 amino acids with the exception of peptide 221-240 which has an overlap of 16 amino acids with peptide 217-240) are shown overlaid.

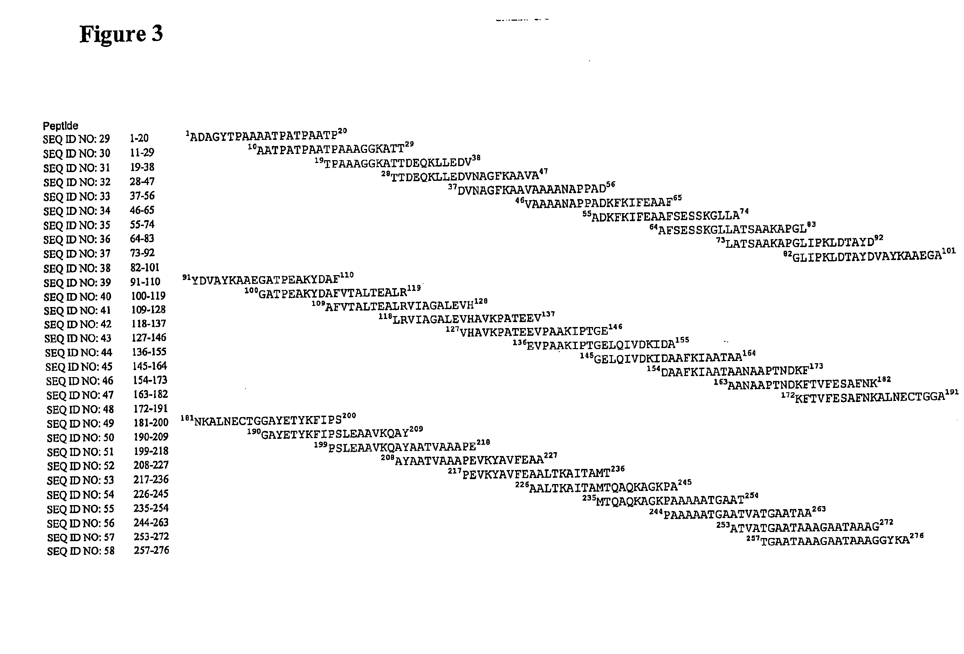

**Figure 3** is a schematic representation of the Lol p 5 20-mer peptide series. The sequence of Lol p 5 is provided in SEQ ID NO:2. The sequence of individual Lol p 5 peptides is shown with the amino acid sequence number designating each peptide given to the left. The overlapping regions of adjacent peptides (spanning 11 amino acids) are shown overlaid.

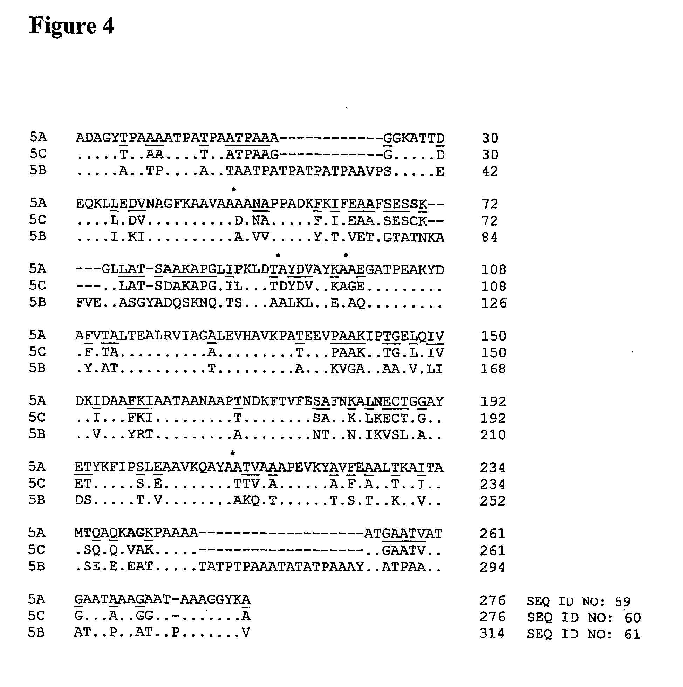

**Figure 4** is a schematic representation of the comparison between the deduced amino acid sequences of three Lol p 5 isoforms. Numbers on the right refer to amino acid residues. Gaps, introduced to maximise homology, are represented by dashes (-). Residues common to all isoforms are represented by dots. Underlined residues of Lol p 5A represent residues common to Lol p 5A and Lol p 5C. Asterisked residues of the Lol p 5A sequence represent residues common to Lol p 5A and Lol p 5B. Bolded residues of the Lol p 5A sequence indicate residues unique to Lol p 5A. Lol p 5A and B sequences are from [Ong *et al,* 1993] and Lol p 5C [Suphioglu *et al,* 1999].

### DETAILED DESCRIPTION OF THE INVENTION

The present invention is predicated, in part, on the identification of Rye grass pollen T cell epitopic regions. The identification of immunodominant epitopes of Rye grass pollen, and in particular Lol p 1 and Lol p 5 has enabled the improvement of diagnostic methodology and the development of therapeutic and prophylactic compositions and treatment approaches for conditions such as, but not limited to, Rye grass pollen allergy.

In accordance with the present invention, overlapping peptides were synthesised based on the Lol p 1 and Lol p 5 amino acid sequences disclosed in SEQ ID NO: 1 and SEQ ID NO: 2, respectively. The T cell immunoreactivity of these peptides is identified in accordance with the present invention on the basis of interactivity of peripheral blood cells or T cells obtained from the peripheral blood of subjects with severe seasonal rhinitis and/or asthma. The identification and generation of these molecules thereby form the basis for a new range of diagnostic, therapeutic and prophylactic reagents and procedures.

Accordingly, one aspect of the present invention provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is any amino acid sequence derived from or homologous to Lol p 1;
and wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

In another aspect of the present invention provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is any amino acid sequence derived from or homologous to Lol p 5;
and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide.

Lol p 1 and Lol p 5 are proteins which have been identified in Rye grass pollen. Accordingly, reference to "Lol p 1" or "Lol p 5" should be understood as including reference to all forms of Lol p 1 or Lol p 5, respectively, or functional derivatives, mutants, homologues or analogues thereof. This includes, for example, all protein forms of Lol p 1 or Lol p 5 or their functional derivatives including, for example, any isoforms which may arise from alternative splicing of Lol p 1 or Lol p 5 mRNA. For instance, "Lol p 1" should be understood to encompass the 4 Lol p 1 or 8 Lol p 5 isoforms described by Smith *et al* (1994). Without limiting the present invention in any way the Lol p 1 isoforms are highly conserved at the amino acid sequence level (Perez *et al.,* 1990; Griffith *et al.,* 1991) whereas the Lol p 5 isoforms are more heterogeneous (Ong *et al.,* 1993; Singh *et al.,* 1991; Suphioglu *et al.,* 1999). Of importance is whether the sequence variations occur at critical residues for MHC binding or T cell receptor restriction. For Lol p 5 peptide 100-119, known sequence variations in the core epitope would not be expected to affect critical residue function (Burton *et al.,* 1999). That such a high frequency of subjects recognise several peptides for each of Lol p 1 and Lol p 5 suggests that sequence variations in general are not important and/or that the different isoforms are presented to and recognised by T cells of the majority of donors. Also included is reference to mutants, polymorphic variants or homologues of Lol p 1 or Lol p 5. It also includes reference to functional analogues of Lol p 1 or Lol p 5 such as may occur where a product which naturally comprises Lol p 1 or Lol p 5 is synthetically generated for the purpose of generating a product The present invention thereby provides epitopes and methods for their use in the diagnosis and treatment of any condition characterised by hypersensitivity to a Lol p 1/Lol p 5 or Lol p 1/Lol p 5-like molecule such as Rye grass pollen allergies or asthma. Preferably, said Lol p 1 or Lol p 5 comprises the sequence set forth in SEQ ID NO: 1 and SEQ ID NO: 2, respectively, or is a functional derivative, homologue, analogue or mutant of said sequence.

The present invention therefore more particularly provides an isolated peptide of the formula:

X₁X₂X₃

wherein:
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 1-240 inclusive of Lol p 1;
and wherein said peptide molecule is capable of interacting with T cells and modifying T cell function when incubated with cells from subj ects having a condition characterised by an aberrant, unwanted or otherwise inappropriate immune response to Lol p 1 or a functional derivative, homologue mutant or analogue of said peptide.

Still more particularly the present invention provides an isolated peptide of the formula:

X₁X₂X₃

wherein
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 19-47, 73-92, 100-128, 127-146, 154-173 or 181-209 inclusive of Lol p 1;
and wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

Still more particularly, X₂ is any amino acid sequence of from 5 to 100 residues derived from, homologous to or contiguous with amino acids 19-38, 28-47, 73-92, 100-119, 109-128, 127-146, 154-173, 181-200, 190-209 inclusive of Lol p 1.

Yet more particularly, X₂ is any amino acid sequence of from 5 to 100 residues derived from homologous to or contiguous with amino acids 19-38, 109-128, 154-173 and/or 190-209 inclusive of Lol p 1.

Most particularly, X₂ is any amino acid sequence of from 5 to 100 residues derived from, homologous to or contiguous with amino acids 19-38 inclusive of Lol p 1.

Still more particularly, X₂ is any amino acid sequence of from 5 to 100 residues derived from, homologous to or contiguous with amino acids 109-128 and/or 154-173 inclusive of Lol p 1.

Still more particularly, X₂ is any amino acid sequence of from 5 to 100 residues derived from, homologous to or contiguous with amino acids 190-209 inclusive of Lol p 1.

The present invention therefore more particularly provides an isolated peptide of the formula:

X₁X₂X₃

wherein:
X₁ and X₃ may be the same or different and each is an amino acid sequence comprising from 0 to 40 naturally or non-naturally occurring amino acid residues:
X₂ is an amino acid sequence of from 5 to 100 residues derived from, homologous to or contiguous with amino acids 1-276 inclusive of Lol p 5;
and wherein said peptide molecule is capable of interacting with T cells and modifying T cell function when incubated with cells from subj ects having a condition characterised by an aberrant, unwanted or otherwise inappropriate immune response to Lol p 5 or a functional derivative, homologue, analogue or mutant of said peptide provided that X₂ is not the amino acid sequence 100-119 or 190-209.

Still more particularly the present invention provides an isolated peptide of the formula:

X₁X₂X₃

wherein
X₁ and X₃ may be the same or different and each is an amino acid sequence comprising from 0 to 40 naturally or non-naturally occurring amino acid residues;
X₂ is an amino acid sequence of from 5 to 100 residues derived from, homologous to or contiguous with amino acids 37-83, 118-137, 145-173, 172-191 or 190-245 inclusive of Lol p 5.
and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide provided that X₂ is not the amino acid sequence 100-119 or 190-209.

Still more particularly, X₂ is any amino acid sequence of from 5 to 100 residues derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.

Yet more particularly, X₂ is any amino acid sequence of from 5 to 100 residues derived from homologous to or contiguous with amino acids 37-56, 145-164, 154-173, 217-236 and/or 226-245 inclusive of Lol p 5.

Most particularly, X₂ is any amino acid sequence of from 5 to 100 residues derived from, homologous to or contiguous with amino acids 37-56 inclusive of Lol p 5.

Still more particularly, X₂ is any amino acid sequence of from 5 to 100 residues derived from, homologous to or contiguous with amino acids 145-164 and/or 154-173 inclusive of Lol p 5.

Still more particularly, X₂ is any amino acid sequence of from 5 to 100 residues derived from, homologous to or contiguous with amino acids 217-236 and/or 226-245 inclusive of Lol p 5.

Reference to "T cells" should be understood as a reference to any cell comprising a T cell receptor. In this regard, the T cell receptor may comprise any one or more of the α, β, γ or δ chains. The present invention is not intended to be limited to any particular functional sub-class of T cells although in a preferred embodiment the subject T cell is a T helper cell and still more preferably a Th2-type cell, predominantly. In this regard, reference to "modifying T cell function" should be understood as a reference to modifying any one or more functions which a T cell is capable of performing. For example, the subject function may be proliferation, differentiation or other form of cellular functional activity such as the production of cytokines. Preferably, the subject functional activity is proliferation.

In terms of modifying the function of T cells from subjects having a condition characterised by an aberrant, unwanted or inappropriate immune response to Lol p 1 or Lol p 5, it should be understood that this is not necessarily a reference to modifying the function of all the T cells in a given sample but is likely, in fact, to reflect the modification or functioning of only some of the T cells in the sample. For example, only a portion of the T helper cells in a given T cell sample may functionally respond to contact with the subject peptide. Such a partial response should be understood to fall within the scope of the present invention. It should also be understood that the T cells which are derived from the subject may be freshly harvested T cells or they may have undergone some form of *in vitro* or *in vivo* manipulation prior to testing. For example, T cell lines may have been generated from the cell sample and it is these T cell lines which then form the subject derived T cell population which is tested in accordance with the present invention. To the extent that the subject functional activity is T cell proliferation, the T cell proliferation assay is preferably performed as disclosed herein. Still more preferably, the subject modification of T cell function is the induction of a proliferation index of >2.5.

Reference to an "aberrant, unwanted or otherwise inappropriate" immune response should be understood as a reference to any form of physiological activity which involves the activation and/or functioning of one or more immune cells where that activity is inappropriate in that it is of an inappropriate type or proceeds to an inappropriate degree. It may be aberrant in that according to known immunological principals it either should not occur when it does so or else should occur when it does not do so. In another example, the immune response may be inappropriate in that it is a physiologically normal response but which is unnecessary and/or unwanted, such as occurs with respect to type-I hypersensitivity responses to innocuous allergens. Preferably said immune response is hypersensitivity to a grass pollen of the family Pooiodeae and even more preferably to Rye grass pollen hypersensitivity or hypersensitivity to immunologically cross-reactive grass pollens of the family Pooiodeae such as Timothy grass pollen.

Reference to "Rye grass pollen hypersensitivity" should be understood to mean the exhibition of clinical symptoms of IgE mediated Rye grass pollen hypersensitivity, for example rhinitis and/or asthma with confirmation of Rye grass specific IgE as determined via skin prick tests to Rye grass pollen extract (wheal diameters ≥ 5mm) and/or using the Kallestad Allercot EAST system (Sanofi-Pasteur Diagnostics, USA) East Score ≥ 3 or other suitable assay for determining allergen specific IgE, such as CAP, Pharmacia. Reference to other Pooiodeae subfamily grass pollen hypersensitivities should be understood to have an analogous definition in terms of these IgE mediated symptoms.

In a preferred embodiment, X₂ comprises not less than about 5 and not greater than about 50 amino acid residues, more preferably not less than about 5 and not greater than about 30 amino acid residues and even more preferably not less than about 5 and not greater than about 20 amino acid residues. In another preferred embodiment, X₂ comprises not less than about 5 and not greater than about 15 amino acid residues. In still another preferred embodiment, X₂ comprises 10, 15, 20, 25 or 30 amino acid residues. Most preferably, X₂ comprises 20 amino acid residues.

In a particularly preferred embodiment, where the subject Rye grass pollen allergen is Lol p 1, X₂ comprises a sequence of at least 5 amino acids derived from one or more of the following amino acid sequences:
LDAKSTWYGKPTGAGPKDNG (SEQ ID NO: 5)
KPTGAGPKDNGGACGYKDVD (SEQ ID NO: 6)
FEIKCTKPESCSGEAVTVTI (SEQ ID NO: 11)
IAPYHFDLSGHAFGSMAKKG (SEQ ID NO: 14)
GHAFGSMAKKGEEQNVRSAG (SEQ ID NO: 15)
AGELELQFRRVKCKYPDDTK (SEQ ID NO: 17)
GSNPNYLAILVKYVDGDGDV (SEQ ID NO: 20)
KGKDKWIELKESWGAVWRID (SEQ ID NO: 23)
KESWGAVWRIDTPDKLTGPF (SEQ ID NO: 24)

More preferably, X₂ comprises a sequence of at least 5 amino acids derived from one or more of SEQ ID NO: 5, SEQ ID NO: 15, SEQ ID NO: 20 and/or SEQ ID NO: 24.

In another preferred embodiment, where the subject Rye grass pollen allergen is Lol p 5, X₂ comprises a sequence of at least 5 amino acids derived from one or more of the following amino acid sequences:
DVNAGFKAAVAAAANAPPAD (SEQ ID NO: 33)
VAAAANAPPADKFKIFEAAF (SEQ ID NO: 34)
ADKFKIFEAAFSESSKGLLA (SEQ ID NO: 35)
AFSESSKGLLATSAAKAPGL (SEQ ID NO: 36)
LRVIAGALEVHAVKPATEEV (SEQ ID NO: 42)
GELQIVDKIDAAFKIAATAA (SEQ ID NO: 45)
DAAFKIAATAANAAPTNDKF (SEQ ID NO: 46)
KFTVFESAFNKALNECTGGA (SEQ ID NO: 48)
PSLEAAVKQAYAATVAAAPE (SEQ ID NO: 51)
AYAATVAAAPEVKYAVFEAA (SEQ ID NO: 52)
PEVKYAVFEAALTKAITAMT (SEQ ID NO: 53)
AALTKAITAMTQAQKAGKPA (SEQ ID NO: 54)

More preferably, X₂ comprises a sequence of at least 5 amino acids derived from one or more of SEQ ID NO: 33, SEQ ID NO: 45, SEQ ID NO: 46, SEQ ID NO: 53 or SEQ ID NO: 54.

Reference to a "peptide" includes reference to a peptide, polypeptide or protein or parts thereof. The peptide may be glycosylated or unglycosylated and/or may contain a range of other molecules fused, linked, bound or otherwise associated to the protein such as amino acids, lipids, carbohydrates or other peptides, polypeptides or proteins. Reference hereinafter to a "peptide" includes a peptide comprising a sequence of amino acids as well as a peptide associated with other molecules such as amino acids, lipids, carbohydrates or other peptides, polypeptides or proteins.

"Derivatives" include fragments, parts, portions and variants from natural, synthetic or recombinant sources including fusion proteins. Parts or fragments include, for example, active regions of the subject peptide. Derivatives may be derived from insertion, deletion or substitution of amino acids. Amino acid insertional derivatives include amino and/or carboxylic terminal fusions as well as intrasequence insertions of single or multiple amino acids. Insertional amino acid sequence variants are those in which one or more amino acid residues are introduced into a predetermined site in the protein although random insertion is also possible with suitable screening of the resulting product. Deletional variants are characterized by the removal of one or more amino acids from the sequence.
Substitutional amino acid variants are those in which at least one residue in the sequence has been removed and a different residue inserted in its place. An example of substitutional amino acid variants are conservative amino acid substitutions. Conservative amino acid substitutions typically include substitutions within the following groups: glycine and alanine; valine, isoleucine and leucine; aspartic acid and glutamic acid; asparagine and glutamine; serine and threonine; lysine and arginine; and phenylalanine and tyrosine. Additions to amino acid sequences include fusions with other peptides, polypeptides or proteins.

Derivatives also include fragments having particular epitopes or parts of the entire protein fused to peptides, polypeptides or other proteinaceous or non-proteinaceous molecules.
For example, α-inhibin or derivative thereof may be fused to a molecule to facilitate its entry into a cell. Analogs of the molecules contemplated herein include, but are not limited to, modification to side chains, incorporating of unnatural amino acids and/or their derivatives during peptide, polypeptide or protein synthesis and the use of crosslinkers and other methods which impose conformational constraints on the proteinaceous molecules or their analogs.

Derivatives of nucleic acid sequences which may be utilised in accordance with the method of the present invention may similarly be derived from single or multiple nucleotide substitutions, deletions and/or additions including fusion with other nucleic acid molecules. The derivatives of the nucleic acid molecules utilised in the present invention include oligonucleotides, PCR primers, antisense molecules, molecules suitable for use in cosuppression and fusion of nucleic acid molecules. Derivatives of nucleic acid sequences also include degenerate variants.

A "variant" or "mutant" of the subject peptide, Lol p 1 or Lol p 5 should be understood to mean molecules which exhibit at least some of the functional activity of the form of peptide, Lol p 1 or Lol p 5 of which it is a variant or mutant. A variation or mutation may take any form and may be naturally or non-naturally occurring.

By "homologue" is meant that the molecule is derived from alternative species.

Analogues contemplated herein include, but are not limited to, modification to side chains, incorporating of unnatural amino acids and/or their derivatives during peptide, polypeptide or protein synthesis and the use of crosslinkers and other methods which impose conformational constraints on the proteinaceous molecules or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carboethoxylation with diethylpyrocarbonate.

Examples of incorporating unnatural amino acids and derivatives during protein synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acids contemplated herein is shown in Table 2.

**TABLE2**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | NmgIn |
| carboxylate | | L-N-methylglutamic acid | Nmglu |
| cyclohexylalanine | Chexa | L-N-metbylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | - L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-omithine | Dom | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl- -aminobutyrate | Mgabu |
| D-α-methylalanine | Dmala | α-methylcyclohexylalanine | Mchexa |
| D-α-methylargmine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylomithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methytnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanin | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl-Nmbc | | | |
| ethylamino)cyclopropane | | | |

Crosslinkers can be used, for example, to stabilise 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety.

Reference to a "functional" derivative, homologue, analogue or mutant should be understood as a reference to a peptide exhibiting one or more of the functional activities of Lol p 1 or Lol p 5 T cell epitope.

It is possible to modify the structure of a peptide according to the invention for various purposes such as for increasing solubility, enhancing therapeutic or preventative efficacy, enhancing stability or increasing resistance to proteolytic degradation. A modified peptide may be produced in which the amino acid sequence has been altered, such as by amino acid substitution, deletion or addition, to modify immunogenicity and/or reduce allergenicity. Similarly components may be added to peptides of the invention to produce the same result.

For example, a peptide can be modified so that it exhibits the ability to induce T cell anergy. In this instance, critical binding residues for the T cell receptor can be determined using known techniques (for example substitution of each residue and determination of the presence or absence of T cell reactivity). In one example, those residues shown to be essential to interact with the T cell receptor can be modified by replacing the essential amino acid with another, preferably similar amino acid residue (a conservative substitution) whose presence is shown to alter T cell reactivity or T cell functioning. In addition, those amino acid residues which are not essential for T cell receptor interaction can be modified by being replaced by another amino acid whose incorporation may then alter T cell reactivity or T cell functioning but does not, for example, eliminate binding to relevant MHC proteins.

Such modifications will result in the production of molecules falling within the scope of "mutants" of the subject peptide as herein defined. "Mutants" should be understood as a reference to peptides which exhibit one or more structural features or functional activities which are distinct from those exhibited by the non-mutated peptide counterpart.

Peptides of the invention may also be modified to incorporate one or more polymorphisms resulting from natural allelic variation and D-amino acids, non-natural amino acids or amino acid analogues may be substituted into the peptides to produce modified peptides which fall within the scope of the invention. Peptides may also be modified by conjugation with polyethylene glycol (PEG) by known techniques. Reporter groups may also be added to facilitate purification and potentially increase solubility of the peptides according to the invention. Other well known types of modification including insertion of specific endoprotease cleavage sites, addition of functional groups or replacement of hydrophobic residues with less hydrophobic residues as well as site-directed mutagenesis of DNA encoding the peptides of the invention may also be used to introduce modifications which could be useful for a wide range of purposes. The various modifications to peptides according to the invention which have been mentioned above are mentioned by way of example only and are merely intended to be indicative of the broad range of modifications which can be effected.

Another aspect of the present invention provides an isolated peptide comprising any amino acid sequence derived from or homologous to Lol p 1 or Lol p 5 wherein said peptide molecule is capable of interacting with T cells and modifying T cell function when incubated with cells from subjects having a condition characterised by an aberrant, unwanted or otherwise inappropriate immune response to Lol p 1 or Lol p 5, respectively, or a functional derivative, homologue, analogue or mutant of said peptide.

More particularly, the present invention provides an isolated peptide comprising an amino acid sequence of from 5-100 residues derived from, homologous to or contiguous with amino acids 1-240 inclusive or derivatives thereof of Lol p 1 wherein said peptide molecule is capable of interacting with T cells and modifying T cell function when incubated with cells from subjects having a condition characterised by an aberrant, unwanted or otherwise inappropriate immune response to Lol p 1 or a functional derivative, homologue, analogue or mutant of said peptide.

In one preferred embodiment said amino acid sequence is derived from, homologous to or contiguous with amino acids 19-47, 73-92, 100-128, 127-146,154-173 or 181-209, inclusive of Lol p 1.

In another preferred embodiment said amino acid sequence is derived from, homologous to or contiguous with amino acids 19-38,28-47, 73-92, 100-119,109-128, 127-146, 154-173, 181-200 and/or 190-209 inclusive of Lol p 1.

In yet another preferred embodiment said amino acid sequence is derived from, homologous to or contiguous with amino acids 19-38, 109-128, 154-173 and/or 190-209 inclusive of Lol p 1.

In another aspect, where the subject Rye grass pollen allergen is Lol p 1, said amino acid sequence comprises a sequence of at least 5 amino acids derived from one or more of the following amino acid sequences:
LDAKSTWYGKPTGAGPKDNG (SEQ ID NO: 5)
KPTGAGPKDNGGACGYKDVD (SEQ ID NO: 6)
FEIKCTKPESCSGEAVTVTI (SEQ ID NO: 11)
IAPYHFDLSGHAFGSMAKKG (SEQ ID NO: 14)
GHAFGSMAKKGEEQNVRSAG (SEQ ID NO: 15)
AGELELQFRRVKCKYPDDTK (SEQ ID NO: 17)
GSNPNYLAILVKYVDGDGDV (SEQ ID NO: 20)
KGKDKWTELKESWGAVWRID (SEQ ID NO: 23)
KESWGAVWRIDTPDKLTGPF (SEQ ID NO: 24)

According to this aspect, said amino acid sequence preferably comprises a sequence of at least 5 amino acids derived from one or more of SEQ ID NO: 5, SEQ ID NO: 15, SEQ ID NO: 20 and/or SEQ ID NO: 24.

In yet another embodiment, the present invention provides an isolated peptide comprising an amino acid sequence of from 5-100 residues derived from, homologoues to or contiguous with amino acids 1-276 inclusive or derivatives thereof of Lol p 5 wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, analogue or mutant of said peptide provided that said amino acid sequence is not the amino acid sequence 100-119 or 190-209.

In one preferred embodiment said amino acid sequence is derived from, homologous to or contiguous with amino acids 37-83, 118-137,145-173, 172-191 and/or 190-245 inclusive of Lol p 5 provided that said amino acid sequence is not the amino acid sequence 100-119 or 190-209.

In another preferred embodiment said amino acid sequence is derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.

In yet another preferred embodiment said amino acid sequence is derived from, homologous to or contiguous with amino acids 37-56, 145-164, 154-173, 217-236 and/or 226-245.

In another aspect, where the subject Rye grass pollen allergen is Lol p 5, said amino acid sequence comprises a sequence of at least 5-amino acids derived from one or more of the following amino acid sequences:
DVNAGFKAAVAAAANAPPAD (SEQ ID NO: 33)
VAAAANAPPADKFKIFEAAF (SEQ ID NO: 34)
ADKFKIFEAAFSESSKGLLA (SEQ ID NO: 35)
AFSESSKGLLATSAAKAPGL (SEQ ID NO: 36)
LRVIAGALEVHAVKPATEEV (SEQ ID NO: 42)
GELQIVDKIDAAFKIAATAA (SEQ ID NO: 45)
DAAFKIAATAANAAPTNDKF (SEQ ID NO: 46)
KFTVFESAFNKALNECTGGA (SEQ ID NO: 48)
PSLEAAVKQAYAATVAAAPE (SEQ ID NO: 51)
AYAATVAAAPEVKYAVFEAA (SEQ ID NO: 52)
PEVKYAVFEAALTKAITAMT (SEQ ID NO: 53)
AALTKAITAMTQAQKAGKPA (SEQ ID NO: 54)

According to this aspect, said amino acid sequence preferably comprises a sequence of at least 5 amino acids derived from one or more of SEQ ID NO: 33, SEQ ID NO: 45, SEQ ID NO: 46, SEQ ID NO: 53 or SEQ ID NO: 54.

The peptides of the present invention may be prepared by recombinant or chemical synthetic means. According to a preferred aspect of the present invention, there is provided a recombinant peptide which is preferentially immunologically reactive with T cells from individuals with Rye Grass pollen hypersensitivity, which is expressed by the expression of a host cell transformed with a vector coding for the peptide sequence of the present invention. The peptide may be fused to another peptide, polypeptide or protein. Alternatively, the peptide may be prepared by chemical synthetic techniques, such as by the Merrifield solid phase synthesis procedure. Furthermore, although synthetic peptides of the formula given above represent a preferred embodiment, the present invention also extends to biologically pure preparations of the naturally occurring peptides or fragments thereof. By "biologically pure" is meant a preparation comprising at least about 60%, preferably at least about 70%, or preferably at least about 80% and still more preferably at least about 90% or greater as determined by weight, activity or other suitable means.

In another aspect it may be particularly useful to generate a mutant peptide comprising T cell epitopic regions but which peptides lack B cell epitopes capable of interacting with IgE. Such peptides may be generated by synthesising peptides comprising only T cell epitopes or by mutating naturally occurring molecules such that the T cell epitopes remain functional while the B cell epitopes are altered to prevent antibody binding.

The present invention should therefore be understood to encompass peptides that comprise at least one B or T cell epitope of Lol p 1 and/or Lol p 5 in conjunction with other amino acids (which may or may not be naturally occurring as amino acid analogues) or other chemical species. In a preferred aspect of the invention such peptides may comprise one or more epitopes of Lol p 1 and/or Lol p 5 , which epitopes may be T or B cell epitopes. Peptides with one or more T cell epitopes of Lol p 1 and/or Lol p 5 are desirable for increased therapeutic effectiveness.

In another aspect, the present invention provides a nucleic acid molecule comprising a sequence of nucleotides encoding or complementary to a sequence encoding the peptides as hereinbefore defined or a derivative, homologue, mutant or analogue thereof. It should be understood that reference to "peptides" includes reference to peptides comprising one or more T cell epitopes. A nucleic acid molecule encoding the subject peptide is preferably a sequence of deoxyribonucleic acids such as cDNA or a genomic sequence. A genomic sequence may comprise exons and introns. A genomic sequence may also include a promoter region or other regulatory regions.

The nucleic acid molecule may be ligated to an expression vector capable of expression in a prokaryotic cell (eg. *E. coli*) or a eukaryotic cell (eg. yeast cells, fungal cells, insect cells, mammalian cells or plant cells). The nucleic acid molecule may be ligated or fused or otherwise associated with a nucleic acid molecule encoding another entity such as, for example, a signal peptide. It may also comprise additional nucleotide sequence information fused, linked or otherwise associated with it either at the 3' or 5' terminal portions or at both the 3' and 5' terminal portions. The nucleic acid molecule may also be part of a vector, such as an expression vector. The latter embodiment facilitates production of recombinant forms of the subject peptide which forms are encompassed by the present invention.

Such nucleic acids may be useful for recombinant production of T cell epitopes of Lol p 1 or Lol p 5 or proteins comprising them by insertion into an appropriate vector and transfection into a suitable cell line. Such expression vectors and host cell lines also form an aspect of the invention.

In producing peptides by recombinant techniques, host cells transformed with a nucleic acid having a sequence encoding a peptide according to the invention or a functional equivalent of the nucleic acid sequence are cultured in a medium suitable for the particular cells concerned. Peptides can then be purified from cell culture medium, the host cells or both using techniques well known in the art such as ion exchange chromatography, gel filtration chromatography, ultrafiltration, electrophoresis or immunopurification with antibodies specific for the peptide.

Nucleic acids encoding Lol p 1 or Lol p 5 or peptides comprising T and/or B cell epitopes of Lol p 1 or Lol p 5 may be expressed in bacterial cells such as *E. coli,* insect cells, yeast or mammalian cells such as Chinese hamster ovary cells (CHO). Suitable expression vectors, promoters, enhancers and other expression control elements are referred to in Sambruck *et al* (1989). Other suitable expression vectors, promotes enhancers and other expression elements are well known to those skilled in the art Examples of suitable expression vectors in yeast include Yep Sec 1 (Balderi *et al.,* 1987); pMFa (Kurjan and Herskowitz, 1982); JRY88 (Schultz *et al.,* 1987) and pYES2 (Invitrogen Corporation, San Diego, CA). These vectors are freely available as are baculovirus and mammalian expression systems. For example, a baculovirus system is commercially available (ParMingen, San Diego, CA) for expression in insect cells while the pMsg vector is commercially available (Pharmacia, Piscataway, NJ) for expression in mammalian cells.

For expression in *E. coli* suitable expression vectors include among others, pTrc (Amann *et al*, 1988) pGex (Amrad Corporation, Melbourne, Australia); pMal (N.E. Biolabs, Beverley, MA); pRit5 (Pharmacia, , Piscataway, NJ); pEt-11d (Novagen, Maddison, WI) (Jameel *et al* 1990) and pSem (Knapp *et al.,* 1990). The use of pTRC, and pEt-11d, for example, will lead to the expression of unfused protein. The use ofpMal, pRit5, pSem and pGex will lead to the expression of allergen fused to maltose E binding protein (pMal), protein A (pRit5), truncated -galactosidase (PSEM) or glutathione S-transferase (pGex). When a T cell epitope of Lol p 1 or Lol p 5 or a peptide comprising it is expressed as a fusion protein, it is particularly advantageous to introduce an enzymatic cleavage site at the fusion junction between the carrier protein and the peptide concerned. The peptide of the invention may then be recovered from the fusion protein through enzymatic cleavage at the enzymatic site and biochemical purification using conventional techniques for purification of proteins and peptides. The different vectors also have different promoter regions allowing constitutive or inducible expression or temperature induction. It may additionally be appropriate to express recombinant peptides in different *E. coli* hosts that have an altered capacity to degrade recombinantly expressed proteins. Alternatively, it may be advantageous to alter the nucleic acid sequence to use codons preferentially utilised by *E. coli,* where such nucleic acid alteration would not effect the amino acid sequence of the expressed proteins.

Host cells can be transformed to express the nucleic acids of the invention using conventional techniques such as calcium phosphate or calcium chloride co-precipitation, DEAE-dextran-mediated transfection or electroporation. Suitable methods for transforming the host cells may be found in (Sambruck *et al.,* 1989), and other laboratory texts. The nucleic acid sequence of the invention may also be chemically synthesised using standard techniques.

In addition to recombinant production of peptides according to the invention, the nucleic acids may be utilised as probes for experimental or purification purposes.

The identification of T cell epitopic regions facilitates the identification and/or rational design of a range of mutant peptide molecules. As detailed hereinbefore, these mutant peptides may comprise one or more mutated B cell epitopes. However there is provided scope for the generation of mutant peptides comprising mutated B cell epitopes or combinations of intact versus mutated B and T cell epitopes. The applications of these molecules are described in more detail below but in a preferred embodiment relate to modulation of the Lol p 1 and/or Lol p 5 hypersensitivity immune response in terms of either a prophylactic or therapeutic treatment

Identification and synthesis of the Lol p 1 and/or Lol p 5 T cell epitopes as disclosed herein now facilitates the development of a range of diagnostic and prophylactic/therapeutic treatment protocols for use with respect to Lol p 1 and/or Lol p 5 related immune conditions. Also facilitated is the development of reagents for use therein. Accordingly, the present invention should be understood to extend to the use of the peptides and monoclonal antibodies or functional derivatives, homologues, analogues or mutants thereof in the therapeutic and/or prophylactic treatment of patients. Such methods of treatment include, but are not limited to:
(i) Administration of the subject peptides to a patient as a means of desensitising or inducing immunological tolerance to Lol p 1/Lol p 5 or Lol p 1/Lol p 5-like molecules. This may be achieved, for example, by inducing Lol p 1 or Lol p 5 directed Th2 anergy or apoptosis. Such an outcome may be achieved by any one of a number of techniques including the use of peptides which maintain T cell epitope reactivity but which either naturally or as a result of mutation are unable to undergo IgE binding. Alternatively, one may utilise desensitisation/treatment protocols which are based on the administration of specific concentrations of a given peptide in accordance with a specific regime in order to induce tolerance. Such methodology may eliminate Lol p 1 and/or Lol p 5 hypersensitivity or it may reduce the severity of Lol p 1 and/or Lol p 5 hypersensitivity.
   Preferably such treatment regimes are capable of modifying the T cell response or both the B and T cell response of the individual concerned. As used herein, modification of the allergic response of the individual suffering from Lol p 1 and/or Lol p 5 hypersensitivity can be defined as inducing either non-responsiveness or diminution in symptoms to the Lol p 1 and/or Lol p 5 molecule as determined by standard clinical procedures (Varney *et al.,* 1990). Diminution in the symptoms includes any reduction in an allergic response in an individual to Lol p 1 and/or Lol p 5 after a treatment regime has been completed. This diminution may be subjective or clinically determined, for example by using standard skin tests known in the art.
   Exposure of an individual to the peptides of the present invention, which peptides comprise at least one T cell epitope, may tolerise or anergise appropriate T cell subpopulations such that they become unresponsive to Lol p 1 and/or Lol p 5 and do not participate in stimulating an immune response upon such exposure.
   Preferably the peptides according to the invention will retain immunodominent T cell epitopes but possess abrogated IgE binding.
   Administration of a peptide of the invention may modify the cytokine secretion profile as compared with exposure to naturally occurring Lol p 1 and/or Lol p 5 allergen. This exposure may also influence T cell subpopulations which normally participate in the allergic response to migrate away from the site or sites of normal exposure to the allergen and towards the site or sites of therapeutic administration. This redistribution of T cell subpopulations may ameliorate or reduce the ability of an individual's immune system to stimulate the usual immune response at the site of normal exposure to the allergen, resulting in diminution of the allergic symptoms.
   Modification of the B cell response may be achieved, for example, via modulation of the cytokine profile produced by T cells, as detailed above. Specifically, decreasing T cell derived IL-4 and IL-13 production thereby decreasing IgE synthesis.
(ii) The peptides of the present invention may be used in the capacity of an adsorbent to remove Lol p 1 and/or Lol p 5 directed T cells from a biological sample or from a patient.

Accordingly, in another aspect the present invention provides a method for the treatment and/or prophylaxis of a condition in a subject, which condition is characterised by the aberrant, unwanted or otherwise inappropriate immune response to Lol p 1 and/or Lol p 5 , said method comprising administering to said subject an effective amount of a peptide as hereinbefore defined for a time and under conditions sufficient to remove or reduce the presence or function in said subject of T cells directed to said Lol p 1 and/or Lol p 5 .

Preferably said condition is hypersensitivity to a grass pollen of the subfamily Pooiodeae and even more preferably Rye grass or Timothy grass pollen hypersensitivity.

An "effective amount" means an amount necessary at least partly to attain the desired immune response, or to delay the onset or inhibit progression or halt altogether, the onset or progression of a particular condition being treated. The amount varies depending upon the health and physical condition of the individual to be treated, the taxonomic group of individual to be treated, the degree of protection desired, the formulation of the composition, the assessment of the medical situation, and other relevant factors. It is expected that the amount will fall in a relatively broad range that can be determined through routine trials.

The subject of the treatment or prophylaxis is generally a mammal such as but not limited to human, primate, livestock animal (e.g. sheep, cow, horse, donkey, pig), companion animal (e.g. dog, cat), laboratory test animal (e.g. mouse, rabbit, rat, guinea pig, hamster), captive wild animal (e.g. fox, deer). Preferably the mammal is a human or primate. Most preferably the mammal is a human.

Reference herein to "treatment" and "prophylaxis" is to be considered in its broadest context. The term "treatment" does not necessarily imply that a subject is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. The term "prophylaxis" may be considered as reducing the severity or onset of a particular condition. "Treatment" may also reduce the severity of an existing condition.

Administration of the peptide of the present invention (herein referred to as "agent") in the form of a pharmaceutical composition, may be performed by any convenient means. The agent of the pharmaceutical composition is contemplated to exhibit therapeutic activity when administered in an amount which depends on the particular case. The variation depends, for example, on the human or animal and the agent chosen. A broad range of doses may be applicable. Considering a patient, for example, from about 0.1 mg to about 1 mg of an agent may be administered per kilogram of body weight per day. Dosage regimes may be adjusted to provide the optimum therapeutic response. For example, several divided doses may be administered daily, weekly, monthly or other suitable time intervals or the dose may be proportionally reduced as indicated by the exigencies of the situation.

The agent may be administered in a convenient manner such as by the oral, intravenous (where water soluble), intraperitoneal, intramuscular, subcutaneous, intradermal, intranasal, sublingual or suppository routes or implanting (e.g. using slow release molecules). The agent may be administered in the form of pharmaceutically acceptable nontoxic salts, such as acid addition salts or metal complexes, e.g. with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder such as tragacanth, corn starch or gelatin; a disintegrating agent, such as alginic acid; and a lubricant, such as magnesium stearate.

In accordance with these methods, the agent defined in accordance with the present invention may be coadministered with one or more other compounds or molecules. By "coadministered" is meant simultaneous administration in the same formulation or in two different formulations via the same or different routes or sequential administration by the same or different routes. By "sequential" administration is meant a time difference of from seconds, minutes, hours or days between the administration of the two types of molecules. These molecules may be administered in any order.

Another aspect of the present invention contemplates the use of an agent as hereinbefore defined in the manufacture of a medicament for the treatment of a condition in a mammal, which condition is characterised by an aberrant, unwanted or otherwise inappropriate immune response to Lol p 1 and/or Lol p 5.

Preferably said condition is hypersensitivity to a grass pollen of the subfamily Pooiodeae and even more preferably Rye grass or Timothy grass pollen hypersensitivity.

In yet another further aspect, the present invention contemplates a pharmaceutical composition comprising an agent as hereinbefore defined and one or more pharmaceutically acceptable carriers and/or diluents. Said agents are referred to as the active ingredients.

The pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit The amount of active compound in such therapeutically useful compositions in such that a suitable dosage will be obtained. Preferred compositions or preparations according to the present invention are prepared so that an oral dosage unit form contains between about 0.1 µg and 2000 mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

The pharmaceutical composition may also comprise genetic molecules such as a vector capable of transfecting target cells where the vector carries a nucleic acid molecule encoding a modulatory agent. The vector may, for example, be a viral vector.

Routes of administration include, but are not limited to, respiratorally (eg. intranasally or orally via aerosol), intratracheally, nasopharyngeally, intravenously, intraperitoneally, subcutaneously, intracranially, intradermally, intramuscularly, intraoccularly, intrathecally, intracereberally, intranasally, infusion, orally, rectally, via IV drip patch and implant Preferably, said route of administration is subcutaneously, intradermally or intranasally.

Yet another aspect of the present invention relates to agents, as hereinbefore defined, when used in the method of the present invention.

In yet another aspect, the present invention should be understood to extend to the use of the peptides of the present invention in diagnostic applications. Said diagnostic applications include, but are not limited to:
(i) To measure the reactivity of a subject's cells to Lol p 1 and/or Lol p 5. This is of use, for example, with respect to the diagnosis and/or monitoring of conditions characterised by an aberrant, unwanted or otherwise inappropriate immune response to Lol p 1 and/or Lol p 5. The peptides may be added into solution or bound to a solid support together with cells derived from peripheral blood or from tissue biopsies either unfractionated, fractionated or derived as a continuous cell line. Reactivity to the subject peptide may then be measured by standard proliferation assays such as incorporation of H³-thymidine, measurement of expressed or secreted molecules such as surface markers, cytokines or other standard assays of cellular activity which are well known in the art.
(ii) The use of T cell epitope comprising peptides together with a T cell proliferation assay which utilises a T cell sample derived from the subject will facilitate, for example, the identification of a T cell responsive population.

Methods of detecting Lol p 1 and/or Lol p 5 may be utilised, for example, to qualitatively or quantitatively detect Lol p 1 and/or Lol p 5 levels. However, these methods may also be utilised to screen for mutations or polymorphisms in Lol p 1 and/or Lol p 5 which mutations may result in, for example, loss of T cell reactivity to Lol p 1 and/or Lol p 5 . These methods may be utilised for the purpose of screening for peptide molecules suitable for use in therapeutically or prophylactically treating an individual suffering from Lol p 1 and/or Lol p 5 related hypersensitivity.

Accordingly, yet another aspect of the present invention is directed to a method of diagnosing or monitoring a condition in a mammal, which condition is characterised by an aberrant, unwanted or inappropriate response to Lol p 1 and/or Lol p 5 , said method comprising screening for Lol p 1 and/or Lol p 5 reactive T cells utilising the peptides hereinbefore defined.

Preferably said condition is hypersensitivity to a grass pollen of the subfamily Pooiodeae and even more preferably Rye grass or Timothy grass pollen hypersensitivity.

In another embodiment the present invention provides diagnostic kits for use in the diagnostic methodology hereinbefore defined.

The present invention will now be further described with reference to the following nonlimiting Examples.

### EXAMPLE 1

### CHARACTERISATION OF THE HUMAN T CELL RESPONSE TO RYE GRASS POLLEN ALLERGENS LOL P 1 AND LOL P 5 MATERIALS AND METHODS

### Subjects

Thirty-eight RGP-sensitive individuals with allergic rhinitis and/or asthma (Johansson SGO J'OB et al. Allergy 56:813-824, 2001) were recruited from the Alfred Hospital Asthma and Allergy Clinic, Melbourne, Australia. Donors were selected on the basis of a history of clinical symptoms of RGP allergy, positive RGP-specific IgE (Kallestad Allercoat Sanofi-Pasteur Diagnostics, EAST score ≥ 3) and positive skin prick test (wheal diameter ≥5 mm). The study was approved by the Alfred Hospital Ethics Committee and informed consent was obtained from all donors before blood was obtained.

### Antigens

Crude RGP (*Lolium perenne*) extract was prepared by gentle mixing of RGP grains (Greer, NC, USA) in PBS at 4°C overnight. The pollen extract was then centrifuged to pellet insoluble material, and the supernatant removed and sterilised by passage through a 0.2 µm filter.

Lol p 1 was purified from crude RGP extracts by SDS-PAGE as previously described (Levy et al., 2001). Lol p 5 was purified by either SDS-PAGE as for Lol p 1 or by affinity chromatography. For this, an anti-Lol p 5 monoclonal antibody (mAb) FMC A7 (Smart *et al.* 1983) conjugated to Sepharose 4B (Pharmacia, Uppsala, Sweden) was used. Briefly, RGP extracts were equilibrated with 10 mM Tris pH 7.5 and passed through the affinity column. Unbound RGP extract was removed by washing with 10 mM Tris pH 7.5. Bound Lol p 5 was then eluted from the column with 0.1 M glycine pH 2.8 and the eluate immediately neutralised with 2 M Tris pH 7.0. The purified Lol p 5 (both SDS-PAGE and affinity purified) was dialysed against PBS and filter sterilised.

Lol p 1 and 5 peptides (20-mers, 11 amino acid overlap) were purchased from Mimotopes, Clayton, Australia. Peptide sequences were based on those published for Lol p 1A (240 amino acids) (Perez et al., 1990) and Lol p 5A (276 amino acids) (Ong et al., 1993).

Mitogenicity of RGP, Lol p 1, Lol p 5 and peptides was excluded by culturing with oligoclonal house dust mite (HDM)- or low ammoniated latex (LAL)-reactive CD4⁺ T cells in the presence of irradiated autologous PBMC, and toxicity was excluded by co-culture of antigens with HDM- or LAL-reactive T cells in the presence of IL-2 (data not shown).

### Generation of short-term RGP-specific TCL

PBMC were cultured in 24-well plates (Costar, MA, USA) at 2.5 x 10⁶ cells/well with RGP extract at a concentration of 25 or 50 µg/ml in RPMI 1640 medium (Gibco BRL, NY, USA) supplemented with 2 mM GlutaMAX I (Gibco BRL), 0.05 mg/ml gentamicin (David Bull Laboratories, Mulgrave, Victoria, Australia) and 5% heat-inactivated human AB⁺ serum (Sigma, MO, USA) for 7 days at 37°C in a 5% CO₂ humidified incubator. At day 7, cells were washed once and restimulated in 24-well plates at 5 x 10⁵ cells/well with 25 or 50 µg/ml of RGP extract in the presence of 5 x 10⁵ cells/well irradiated (3000 rads) autologous PBMC as a source of antigen presenting cells (APC). At day 2 following restimulation, 10 U/ml of Lymphocult T-LF (LC; Biotest, Germany) was added as a source of IL-2 and at day 4, 1 ml of culture medium was removed and replaced with fresh medium and 10 U/ml of LC. For some experiments due to low cell numbers at 2 weeks, 3 week TCL were generated by restimulation with antigen and LC as above for a further week. In all experiments T cells were rested for 6 to 7 days after the last addition of antigen and APC before testing.

### TCL proliferation assays

T cells (5 x 10⁴/well) were cultured in 96-well U bottom plates with either RGP extract at concentrations ranging from 25 to 200 µg/ml, purified Lol p 1 (0.12 to 2 µg/ml) or Lol p 5 (0.1 to 10 µg/ml), or Lol p 1 or Lol p 5 peptides (10 µg/ml), or medium alone (to determine background levels of proliferation), in the presence of washed autologous irradiated (3000 rads) PBMC (5 x 10⁴/well). For some TCL, there were insufficient cells to permit testing to both Lol p 1 and Lol p 5 peptide series. Cultures were incubated for 3 days, pulsed for the last 16 hours with ³H-thymidine (1 µCi/well) and harvested onto glass fibre filters. ³H-thymidine incorporation was measured by liquid scintillation spectroscopy and the mean counts per minute (cpm) for triplicate cultures was determined. Responses were considered positive if the stimulation index (SI, cpm of antigen-stimulated T cells divided by cpm of unstimulated T cells) was ≥ 2.5. Where several concentrations of allergen were tested (i.e., RGP, Lol p 1 and Lol p 5), values corresponding to the highest proliferation are shown.

### Cytokine detection in culture supernatants

Supernatants of triplicate cultures were harvested and pooled from T cell proliferation assay cultures at 48 hours for the detection of IL-5 and IFN-γ production by sandwich ELISA. Briefly white Maxisorp (NUNC, Roskilde, Denmark) ELISA plates were coated with capture mAb (anti-IL-5, Pharmingen, San Diego, CA, USA; anti-IFN-γ, Endogen, Woburn, MA, USA) overnight at 4°C. Plates were then washed and wells blocked with 1 % Bovine Serum Albumin/PBS for 1 hour at room temperature. Following washing, test samples (neat) and serial dilutions of recombinant human IL-5 (PharMingen, USA) or IFN-γ (Endogen, USA) (for the construction of standard curves) were added and incubated overnight at 4°C. Following washing, plates were incubated with biotinylated detecting mAb (anti-IL-5, Pharmingen, USA; anti-IFN-γ, Endogen, USA) for 1 hour at room temperature. Plates were then washed and incubated with streptavidin-peroxidase (Amersham, Piscataway, NJ, USA) for 30 minutes at room temperature. Following washing, a chemiluminescent substrate (Du Pont, Wilmington, DE, USA) was added to each well and plates read in a Lumicount microplate Glow Luminometer (Packard Instrument Company, Meriden, CT, USA), 0.5 seconds/well, automatic sensitivity setting. Standard curve construction and determination of test sample cytokine levels were performed using Packard I-smart software. The limits of detection for IL-5 and IFN-γ were 10 pg/ml and 2 pg/ml respectively.

### EXAMPLE 2

### T CELL RESPONSE TO RGP ALLERGENS

Stimulation of TCL with crude RGP extract induced good proliferation responses in all except two TCL (Tables 3 and 4). For these two TCL, the stimulation index was below the arbitrary cut-off of 2.5. In both these cases Lol p 5 peptide reactivity was demonstrated suggesting that the poor response to crude RGP extract may have been due to the use of a suboptimal dose. Only one concentration of RGP was used for these subjects due to limited cell numbers. Ten of fourteen (71%) TCL tested responded to purified Lol p 1 and nine of fifteen (60%) TCL responded to purified Lol p 5. However, if allergen responsiveness was considered in terms of response to allergen or peptide, 79% of TCL responded to Lol p 1 and 91 % to Lol p 5. These results demonstrate the importance of these two allergens at the T cell level. The fine specificity of Lol p 1 and Lol p 5 reactive T cells was revealed by comparison of responses to the nested sets of peptides.

### EXAMPLE 3

### TCL RESPONSES TO LOL P 1 PEPTIDES

Twenty four of 34 (71 %) RGP-specific TCL responded to one or more Lol p 1 peptides. The proliferative responses of peptide-responsive RGP-specific TCL are shown in Table 3. With the exception of four Lol p 1 peptides (55-74, 163-182, 199-218 and 208-227) all were recognised by one or more TCL. When a responder frequency analysis was performed, four peptides were recognised by 25% or more Lol p 1 peptide-responsive donors (Fig. 1). These peptides encompassed regions 19-38, 109-128, 154-173 and 190-209 of the Lol p 1 amino acid sequence. All four peptides were distinct regions of Lol p 1 indicating that each contains separate and discrete T cell epitopes. The highest frequency of donor recognition of a single peptide was 46% for Lol p 1 (19-38). Taken together the four most frequently recognised Lol p 1 peptides were recognised by 75% of Lol p 1 peptide-responsive RGP-specific TCL.

### EXAMPLE 4

### TCL RESPONSES TO LOL P 5 PEPTIDES

T cell responsiveness to Lol p 5 peptides was detected in 15 of 21 (71%) of TCL tested, with determinants spread throughout the central region of the molecule (Table 4). No donors responded to peptides encompassing the N- and C-terminus of Lol p 5 (residues 1-36 and 246-276 respectively), nor were T cell responses detected towards peptides 82-101, 91-110, 109-128, 127-146 or 136-155. Of the 30 overlapping Lol p 5 peptides only 17 (57%) were recognised by one or more RGP-specific TCL. As observed for the TCL responses to Lol p 1 peptides, several TCL responded to adjacent overlapping peptides suggesting that the responses may either be to separate T cell epitopes or to a common T cell epitope encompassed by both peptides. Of the 17 positively recognised peptides, seven were recognised by more than 25% of the peptide responsive TCL, these being peptides 37-56, 100-119, 145-164, 154-173, 190-209, 217-236 and 226-245 (Fig. 1). The peptides to which the highest frequency of responses were directed were peptides 37-56 and 154-173, each being recognised by more than 50% of peptide-responsive donors. Together these two peptides were recognised by 80% of peptide-responsive donors. For the seven dominant Lol p 5 peptides, 93% of peptide-responsive donors recognised one or more of these peptides, however the same recognition frequency could also be achieved with only four (37-56, 100-119, 154-173, 217-236) of these seven dominant peptides.

### EXAMPLE 5

### PRODUCTION OF IL-5 AND IFN-γ BY RGP-SPECIFIC TCL

The production of IL-5 and IFN-γ was tested in 12 RGP-specific TCL (Table 5). The IL-5 and IFN-γ responses were determined for selected T cell reactive and non-reactive peptides based on proliferation assays. Following stimulation with T cell reactive Lol p 1 peptides cytokine profiles varied, with some peptides inducing the production of both IL-5 and IFN-γ and others inducing the secretion of IFN-γ only. T cell reactive Lol p 5 peptides generally induced the production of both cytokines with higher levels of IL-5. IL-5 was rarely detected in supernatants from cultures that were incubated with peptides that failed to stimulate T cell proliferation and similarly the levels of IFN-γ production in these cultures were minimal or undetectable.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Alexander C, Oldfield WLG, Shirley K, Larche M, Kay AB. 2001, J Allergy Clin Immunol; 102:S217.
Amann et al., 1998, Gene., 69:301-315
Balderi et al., 1987, Embo J., 6:229-234
Blaher B, Suphioglu C, Knox RB, Singh MB, McCluskey J, Rolland JM. 1996, J Allergy Clin Immunol; 98:124-132.
Bousquet J, Lockey RF, Malling HJ. 1998, WHO Position Paper; Allergy; 53:1-42.
Bungy GA, Rodda S, Roitt I, Brostoff J. 1994, Eur J Immunol; 24:2098-2103.
Bungy Poor Fard GA, Latchman Y, Rodda S, Geysen M, Roitt I, Brostoff J. 1993, Clin Exp Immunol; 94:111-116.
Burton *et al,* 1999
de Lalla C, Sturniolo T, Abbruzzese L, et al. 1999, J Immunol; 163:1725-1729.
Ford SA, Baldo BA. 1986, Int Arch Allergy Appl Immunol; 81:193-203.
Jameel et al., 1990, J. Virol., 64:3963-3966
Johansson SGO J'OB, Bousquet, J., Bruijnzeel-Koomen, C. 2001, et al. Allergy 56:813-824
Knapp et al., 1990, Bio Techniques., 8:280-281
Kurjan and Herskowitz., 1982, Cell., 30:933-943
Levy D, Davies J, O'Hehir R, Suphioglu C. 2001, Electrophoresis; 22:1900-1905.
Miyamoto T: Advances in Allergy and Clinical Immunology. Godard P, Bousquet J, Michel FB (eds) pp343-347. The Parthenon Publishing Group, Cornforth, UK, 1992.
Muller U, Akdis CA, Fricker M, et al. 1998 J Allergy Clin Immunol; 101:747-754.
Oldfield WLG, K. S, M. L, A.B. K. 2001, J Allergy Clin Immunol; 107:S216.
Ong EK, Griffith IJ, Knox RB, Singh MB. 1993, Gene; 134:235-240.
Pene J, Desroches A, Paradis L, et al. 1998, J Allergy Clin Immunol; 102:571-578.
Perez M, Ishioka GY, Walker LE, Chesnut RW. 1990, J Biol Chem; 265:16210-16215.
Rolland J, O'Hehir R. 1998, Curr Opin Immunol; 10:640-645.
Romagnani S. 1997, Immunol Today 1997; 18:263-266.
Sambruck et al., 1989, Cold Spring Harbour Laboratory Press; Cold Spring Harbour, New York
Schramm G, Kahlert H, Suck R, et al. 1999 J Immunol; 162:2406-2414.
Schultz et al., 1987, Gene., 54:113-123
Smart *et al.* 1983
Smith AM, Chapman MD, Taketomi EA, Platts-Mills TA, Sung SS. 1998, J Allergy Clin Immunol; 101:423-425.
Spiegelberg HL, Beck L, Stevenson DD, Ishioka GY. 1994, J Immunol; 152:4706-4711.
Stewart GA, Turner KJ, Baldo BA, et al. 1988, Int Arch Allergy Appl Immunol; 86:9-18.
Varney MD, Tait BD. 1998, Eur. J. Immunogenetics., 25:371-374
Varnet et al. 1990 British Medical Journal 302:265-269.
Vrtala S, Hirtenlehner K, Susani M, et al. 1999, Int Arch Allergy Immunol; 118:218-219.

**Table 3 Lol p 1 epitope specificities of RGP oligoclonal TCLs**

| Subject | | | | | | | | | | | | | | | | | | | | | | | | |
|---|---|---|---|---|---|---|---|---|---|---|---|---|---|---|---|---|---|---|---|---|---|---|---|---|
| Peptide | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
| 1-20 | | | | | | | | | | | | | | | | | | | | | | | | 2.6 |
| 10-29 | | | | 3.1 | | | | | | | | | 3.0 | | | | | | | | | | | |
| 19-38 | | 5.2 | 3.3 | 12.3 | | 2.7 | 3.2 | 3.8 | | | | 2.5 | | | 10.5 | | 5.3 | | 10.3 | | 3.8 | | | |
| 28-47 | | | | 4.1 | | | | | | | 3.5 | | | | 2.6 | | | | 3.0 | | | | | |
| 37-56 | | | | 2.5 | | | | | | | | | | | | | | | | 2.6 | | | | |
| 46-65 | | | | | 8.4 | | | | | | | 4.0 | | | | | | | | | | | | |
| 55-74 | | | | | | | | | | | | | | | | | | | | | | | | |
| 64-83 | 7.2 | | | | | | | | | | | | | | 3.5 | | | | | | | | | |
| 73-92 | 3.9 | | | 2.8 | | | | | | | 2.6 | | | | | | | | | | | | | |
| 82-101 | | | | | | | | | | | 3.6 | | | | | | | | | | | | | |
| 91-110 | | | | 3.6 | | | | | | | 4.9 | | | | | | | | | | | | | |
| 100-119 | | 9.4 | | 6.8 | | | | | | | 7.9 | | | | 9.2 | | | | 3.7 | | | | | |
| 109-128 | | 12.9 | 2.8 | | | | | | | | | | | | 7.1 | 3.0 | 2.7 | | 120 | | | | | |
| 118-137 | | 6.2 | | | | | | | | | | 2.5 | | | | | | | | | | | | |
| 127-146 | | 5.8 | | | | | | | | | | | 3.7 | | | | 3.0 | | | | | | | |
| 136-155 | | | | 2.5 | | | | | | | | | | 4.4 | | | | | | | | | | |
| 145-164 | | | | | | | | | | | | 4.5 | | | | | | | | | | | | |
| 154-173 | | | | 3.0 | | | | | 3.0 | | 4.2 | 3.0 | | | 3.1 | | | | | | | | 4.2 | |
| 163-182 | | | | | | | | | | | | | | | | | | | | | | | | |
| 172-191 | | | | | | | | | 25 | | | | | | | | | | | | | | | |
| 181-200 | | | | 4.5 | | | | | | | 7.3 | | | | 5.8 | | | 2.6 | | | | | 6.2 | |
| 190-209 | | | | 6.2 | | | | | | 3.7 | 3.7 | | | | 5.1 | | | 5.4 | 3.0 | | | 5.7 | 5.2 | |
| 199-218 | | | | | | | | | | | | | | | | | | | | | | | | |
| 208-227 | | | | | | | | | | | | | | | | | | | | | | | | |
| 217-236 | | | | | | | | | | | 4.4 | | | | | | | | | | | | | |
| 221-240 | | | | | | | | | | | 3.3 | | | | | | | | | | | | | |
| RGP | 9.7 | 9.3 | 14.4 | 22.1 | 9.9 | 15.3 | 4.0 | 6.8 | 7.0 | 5.6 | 10.0 | 3.5 | 3.0 | 5.6 | 20.2 | 12.9 | 19.3 | 3.9 | 19.7 | 19.1 | 30.5 | 20.0 | 15.2 | 14.2 |
| Lol p 1 | 5.9 | 3.0 | NT | 2.0 | 32.5 | 4.0 | | NT | | NT | NT | NT | NT | NT | 3.3 | NT | | | 10.5 | NT | NT | NT | NT | NT |

| | | | | | | | | | | | | | | | | | | | | | | | | |
|---|---|---|---|---|---|---|---|---|---|---|---|---|---|---|---|---|---|---|---|---|---|---|---|---|
| Proliferative responses for Lol p 1 peptide-responsive TCL are shown as stimulation indices. Only responses considered positive, **i.e.,** where stimulation indices were 2.5 or greater, are shown. Background responses of T cells cultured with irradiated APC in the absence of antigen were 2121 ± 2628 cpm (mean ± SD). The maximal RGP and Lol p 1 response is shown for each donor. NT, not tested. | | | | | | | | | | | | | | | | | | | | | | | | |

**Table 4 Lol p 5 peptide specificities of RGP oligoclonal TCLs**

| Subject | | | | | | | | | | | | | | | |
|---|---|---|---|---|---|---|---|---|---|---|---|---|---|---|---|
| Peptide | 2 | 4 | 6 | 7 | 8 | 11 | 12 | 13 | 20 | 27 | 29 | 35 | 36 | 37 | 38 |
| 1-20 | | | | | | | | | | | | | | | |
| 10-29 | | | | | | | | | | | | | | | |
| 19-38 | | | | | | | | | | | | | | | |
| 28-47 | | | | | | | | | | | | | | | |
| 37-56 | 4.3 | | 2.7 | | | 4.6 | | 3.9 | 4.9 | | 2.8 | 4.1 | | | 4.0 |
| 46-65 | | | | | | | | | | | | | 2.7 | 6.6 | |
| 55-74 | | | | | | | | | | | | | 3.3 | | 3.3 |
| 84-83 | | 2.8 | | 2.8 | | | | | | | | | | | |
| 73-92 | | | | 2.6 | | | | | | | | | | | |
| 82-101 | | | | | | | | | | | | | | | |
| 91-110 | | | | | | | | | | | | | | | |
| 100-119 | | 3.8 | | | | 10.0 | 7.5 | | | | | 2.5 | | | |
| 109-128 | | | | | | | | | | | | | | | |
| 118-137 | | | | | | | | | 4.5 | 2.9 | | | | | |
| 127-146 | | | | | | | | | | | | | | | |
| 136-155 | | | | | | | | | | | | | | | |
| 145-164 | | 3.1 | | | | | | 6.4 | 5.3 | | 4.5 | 5.3 | | | |
| 154-173 | 3.8 | 4.6 | | | 5.2 | 4.5 | | | | 2.5 | | 4.1 | | 6.4 | 4.5 |
| 163-182 | | | | | | | | | 2.5 | | | | | | |
| 172-191 | | 2.7 | | | | | 2.5 | | 3.0 | | | | | | |
| 181-200 | | | | | | | | | 3.4 | | | | | | |
| 190-209 | 3.0 | 5.1 | | | | 4.0 | | | | | 3.1 | | | | |
| 199-218 | | 2.5 | | | | 5.6 | | | | | | | | 4.8 | |
| 208-227 | | | | | | 4.3 | | | 4.6 | | | | 2.6 | | |
| 217-236 | | | | | | 5.4 | | | 4.5 | 9.5 | | 3.5 | 3.5 | 11.8 | |
| 226-245 | 3.3 | | | | | 3.5 | | 2.9 | | 7.1 | | | | | |
| 235-254 | | | | | | | | | | | | | | | |
| 244-263 | | | | | | | | | | | | | | | |
| 253-272 | | | | | | | | | | | | | | | |
| 257-276 | | | | | | | | | | | | | | | |
| RGP | | 4.9* | | | 4.3† | 9.4† | | NT | NT | 3.8† | NT | NT | NT | NT | 3.2* |
| Lol p 1 | 5.7 | 5.2 | 19.3 | 3.0 | 12.3 | 12.8 | 7.0 | | 4.8 | 3.2 | 7.8 | | 7.6 | 3.6 | 5.3 |

| | | | | | | | | | | | | | | | |
|---|---|---|---|---|---|---|---|---|---|---|---|---|---|---|---|
| Proliferative responses for Lot p 5 peptide-responsive TCL are shown as stimulation indices. Only responses considered positive, i.e., where stimulation indices were 2.5 or greater, are shown. Background responses of T cells cultured with irradiated APC in the absence of antigen were 2720 ± 2971 cpm (mean ± SD). The maximal RGP and Lol p 5 response is shown for each donor. NT, not tested. ⁻SDS-PAGE purified Lol p 5. †Affinity purified Lol p5. | | | | | | | | | | | | | | | |

**Table 5 IL-5 and IFN-y production by RGP-specific TCL stimulated with Lol p 1 or Lol p 5 peptides**

| | | | | *Non-proliferation inducing peptides* | |
|---|---|---|---|---|---|
| | *Proliferation inducing peptides* | | | | |
| *Subject* | *Peptide* | *IL-5 (pg*/*ml)* | *IFN-γ (pg*/*ml)* | *IL-5 (pg*/*ml)* | IFN-γ (pg/ml) |
| *Lol p 1 peptides* | | | | | |
| 3 | 19-38 | UD | 20.9 | UD | 5.7 |
| | 109-128 | UD | 32.1 | | |
| 10 | 190-209 | UD | 9.0 | UD | 5.4 |
| 14 | 136-155 | UD | 106.4 | UD | 24.9 |
| 15 | 19-38 | 182.7 | 204.1 | UD | 24.5 |
| | 28-47 | UD | 23.2 | | |
| | 64-83 | 26.5 | 38.8 | | |
| | 100-119 | 275.6 | 167.1 | | |
| | 109-128 | 160.7 | 143.3 | | |
| | 154-173 | 26.6 | 83.9 | | |
| | 181-200 | 13.3 | 56.6 | | |
| | 190-209 | 19.3 | 63.5 | | |
| 16 | 109-128 | UD | 13.8 | UD | 5.1 |
| 19 | 19-38 | UD | 42.8 | UD | 0.6 |
| | 28-47 | UD | 5.2 | | |
| | 100-119 | UD | UD | | |
| | 109-128 | UD | 8.3 | | |
| | 190-209 | 14.7 | UD | | |
| 20 | 37-56 | 46.9 | 11.1 | UD | 5.9 |
| 23 | 154-173 | 12.4 | 11.2 | 3.9 | 4.6 |
| | 181-200 | 25.1 | 11.7 | | |
| | 190-209 | UD | 8.3 | | |
| 24 | 1-20 | 46.1 | UD | UD | UD |
| Lol p 5 peptides | | | | | |
| 2 | 37-56 | 692.8 | 317.0 | UD | 38.3 |
| | 154-173 | 546.3 | 400.3 | | |
| | 190-209 | 518.1 | 87.8 | | |
| | 226-245 | UD | 292.0 | | |
| 38 | 37-56 | 275.1 | 85.0 | 12.2 | 27.6 |
| | 55-74 | 220.7 | 54.2 | | |
| | 154-173 | 256.3 | 198.1 | | |

| | | | | | |
|---|---|---|---|---|---|
| Supernatants were harvested at 48 h from T cell cultures and tested by ELISA for the presence of IL-5 and IFN-γ. Cytokine levels induced by immunoreactive peptides are shown en the left of the table and as mean values for unreactive peptides on the right. UD, undetectable. | | | | | |

### FURTHER EMBODIMENTS OF THE INVENTION

The invention also provides the following:
I. An isolated peptide of the formula:

   X₁ X₂ X₃

   wherein:
   X₁ and X₃ may be the same or different and each is an amino acid sequence comprising from 0 to 40 naturally or non-naturally occurring amino acid residues;
   X₂ is any amino acid sequence derived from or homologous to Lol p 1, and wherein said peptide molecule is capable of interacting with T cells and modifying T cell function when incubated with cells subjects having a condition characterised by an aberrant, unwanted or otherwise inappropriate immune response to Lol p 1 or a functional derivative, homologue, mutant or analogue of said peptide.
II. An isolated peptide of the formula:

   X₁ X₂ X₃

   wherein:
   X₁ and X₃ may be the same or different and each is an amino acid sequence comprising from 0 to 40 naturally or non-naturally occurring amino acid residues;
   X₂ is any amino acid sequence derived from or homologous to Lol p 5, and wherein said peptide molecule is capable of interacting with T cells and modifying T cell function when incubated with cells from subjects having a condition characterised by an aberrant, unwanted or otherwise inappropriate immune response to Lol p 5 or a functional derivative, homologue, mutant or analogue of said peptide provided that X₂ is not the amino acid sequence 100-119 or 190-209.
III. The peptide according to item I wherein X₂ is an amino acid sequence of from 5 to 100 residues derived from, homologous to or contiguous with amino acids 1-240 inclusive of Lol p 1.
IV. The peptide according to item III wherein said amino acid sequence is derived from, homologous to or contiguous with amino acids 19-47, 73-92, 100-128, 127-146, 154-173 and/or 181-209 inclusive of Lol p 1.
V. The peptide according to item IV wherein said amino acid sequence is derived from, homologous to or contiguous with amino acids 19-38, 28-47, 73-92, 100-119, 109-128, 127-146, 154-173, 181-200 and/or 190-209 inclusive of Lol p 1.
VI. The peptide according to item V wherein said amino acid sequence is derived from, homologous to or contiguous with amino acids 19-38, 109-128, 154-173 and/or 190-209 inclusive of Lol p 1.
VII. The peptide according to item VI wherein said amino acids are 19-38 inclusive of Lol p 1.
VIII. The peptide according to item VI wherein said amino acids are 109-128 and/or 154-173 inclusive of Lol p 1.
IX. A peptide according to item VI wherein said amino acids are 190-209 inclusive of Lol p 1.
X. The peptide according to item II wherein X₂ is an amino acid sequence of from 5 to 100 residues derived from, homologous to or contiguous with amino acids 1-276 inclusive of Lol p 5.
XI. The peptide according to item II wherein said amino acid sequence is derived from, homologous to or contiguous with amino acids 37-83, 118-137, 145-173, 172-191 or 190-245 inclusive of Lol p 5.
XII. The peptide according to item XI wherein said amino acid sequence is derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.
XIII. The peptide according to item XII wherein said amino acid sequence is derived from, homologous to or contiguous with amino acids 37-56, 145-164, 154-173, 217-236 and/or 226-245 inclusive of Lol p 5.
XIV. The peptide according to item XIII wherein said amino acids are 37-56 inclusive of Lol p 5.
XV. The peptide according to item XIII wherein said amino acids are 145-164 and/or 154-173 inclusive of Lol p 5.
XVI. A peptide according to item XIII wherein said amino acids are 217-236 and/or 226-245 inclusive of Lol p 5.
XVII. The peptide according to item III wherein said amino acid sequence comprises at least 5 amino acids derived from one or more of the following amino acid sequences:
   LDAKSTWYGKPTGAGPKDNG (SEQ ID NO: 5)
   KPTGAGPKDNGGACGYKDVD (SEQ ID NO: 6)
   FEIKCTKPESCSGEAVTVTI (SEQ ID NO: 11)
   IAPYHFDLSGHAFGSMAKKG (SEQ ID NO: 14)
   GHAFGSMAKKGEEQNVRSAG (SEQ ID NO: 15)
   AGELELQFRRVKCKYPDDTK (SEQ ID NO: 17)
   GSNPNYLAILVKYVDGDGDV (SEQ ID NO: 20)
   KGKDKWIELKESWGAVWRID (SEQ ID NO: 23)
   KESWGAVWRIDTPDKLTGPF (SEQ ID NO: 24)
XVIII. The peptide according to item XVII wherein said amino acid sequence is derived from one or more of SEQ ID NO: 5, SEQ ID NO: 15, SEQ ID NO: 20 or SEQ ID NO: 24.
XIX. The peptide according to item X wherein said amino acid sequence comprises at least 5 amino acids derived from one or more of the following amino acid sequences:
   DVNAGFKAAVAAAANAPPAD (SEQ ID NO: 33)
   VAAAANAPPADKFKIFEAAF (SEQ ID NO: 34)
   ADKFKIFEAAFSESSKGLLA (SEQ ID NO: 35)
   AFSESSKGLLATSAAKAPGL (SEQ ID NO: 36)
   LRVIAGALEVHAVKPATEEV (SEQ ID NO: 42)
   GELQIVDKIDAAFKIAATAA (SEQ ID NO: 45)
   DAAFKIAATAANAAPTNDKF (SEQ ID NO: 46)
   KFTVFESAFNKALNECTGGA (SEQ ID NO: 48)
   PSLEAAVKQAYAATVAAAPE (SEQ ID NO: 51)
   AYAATVAAAPEVKYAVFEAA (SEQ ID NO: 52)
   PEVKYAVFEAALTKAITAMT (SEQ ID NO: 53)
   AALTKAITAMTQAQKAGKPA (SEQ ID NO: 54)
XX. The peptide according to item XIX wherein said amino acid sequence is derived from one or more of SEQ ID NO: 33, SEQ ID NO: 45, SEQ ID NO: 46, SEQ ID NO: 53 or SEQ ID NO: 54.
XXI. An isolated peptide comprising an amino acid sequence derived from or homologous to Lol p 1 or Lol p 5 wherein said peptide molecule is capable of interacting with T cells and modifying T cell function when incubated with cells from subjects having a condition characterised by an aberrant, unwanted or otherwise inappropriate immune response to Lol p 1 or Lol p 5 or a functional derivative, homologue, analogue or mutant of said peptide.
XXII. The peptide according to item XXI wherein said amino acid sequence is of 5-100 residues derived from, homologous to or contiguous with amino acids 1-240 inclusive of Lol p 1.
XXIII. The peptide according to item XXII wherein said amino acid sequence is derived from, homologous to or contiguous with amino acids 19-47, 73-92, 100-128, 127-146, 154-173 and/or 181-209 inclusive of Lol p 1.
XXIV. The peptide according to item XXIII wherein said amino acid sequence is derived from, homologous to or contiguous with amino acids 19-38, 28-47, 73-92, 100-119, 109-128, 127-146, 154-173, 181-200 and/or 190-209 inclusive of Lol p 1.
XXV. The peptide according to item XXIV wherein said amino acid sequence is derived from, homologous to or contiguous with amino acids 19-38, 109-128, 154-173 and/or 190- 209 inclusive of Lol p 1.
XXVI. The peptide according to item XXV wherein said amino acids are 19-38 inclusive of Lol p 1.
XXVII. The peptide according to item XXV wherein said amino acids are 109-128 and/or 154-173 inclusive of Lol p 1.
XXVIII. The peptide according to item XXV wherein said amino acids are 190-209 inclusive of Lol p 1.
XXIX. The peptide according to item XXV wherein said amino acid sequence comprises at least 5 amino acids derived from one or more of the following amino acid sequences:
   LDAKSTWYGKPTGAGPKDNG (SEQ ID NO: 5)
   KPTGAGPKDNGGACGYKDVD (SEQ ID NO: 6)
   FEIKCTKPESCSGEAVTVTI (SEQ ID NO: 11)
   IAPYHFDLSGHAFGSMAKKG (SEQ ID NO: 14)
   GHAFGSMAKKGEEQNVRSAG (SEQ ID NO: 15)
   AGELELQFRRVKCKYPDDTK (SEQID NO: 17)
   GSNPNYLAILVKYVDGDGDV (SEQ ID NO: 20)
   KGKDKWIELKESWGAVWRID (SEQ ID NO: 23)
   KESWGAVWRIDTPDKLTGPF (SEQ ID NO: 24)
XXX. The peptide according to item XXIX wherein said amino acid sequence is derived from one or more of SEQ ID NO: 5 or SEQ ID NO: 15, SEQ ID NO: 20 or SEQ ID NO: 24.
XXXI. The peptide according to item XXI wherein said amino acid sequence is of 5-100 residues derived from, homologous to or contiguous with amino acids 1-276 inclusive of Lol p 5 provided that said peptide does not consist of the amino acid sequence 100-110 or 190-209.
XXXII. The peptide according to item XXXI wherein said amino acid sequence is derived from, homologous to or contiguous with amino acids 37-83, 118-137, 145-173, 172-191 or 190-245 inclusive of Lol p 5.
XXXIII. The peptide according to item XXXII wherein said amino acid sequence is derived from, homologous to or contiguous with amino acids 37-56, 46-65, 55-74, 64-83, 118-137, 145-164, 154-173, 172-191, 199-218, 208-227, 217-236 and/or 226-245 inclusive of Lol p 5.
XXXIV. The peptide according to item XXXIII wherein said amino acid sequence is derived from, homologous to or contiguous with amino acids 37-56, 145-164, 154-173, 217-236 and/or 226-245 inclusive of Lol p 5.
XXXV. The peptide according to item XXXIV wherein said amino acids are 37-56 inclusive of Lol p 5.
XXXVI. The peptide according to item XXXIV wherein said amino acids are 145-164 and/or 154-173 inclusive of Lol p 5.
XXXVII. The peptide according to item XXXIV wherein said amino acids are 217-236 and/or 226-245 inclusive of Lol p 5.
XXXVIII. The peptide according to item XXXIV wherein said amino acid sequence comprises at least 5 amino acids derived from one or more of the following amino acid sequences:
   DVNAGFKAAVAAAANAPPAD (SEQ ID NO: 33)
   VAAAANAPPADKFKIFEAAF (SEQ ID NO: 34)
   ADKFKIFEAAFSESSKGLLA (SEQ ID NO: 35)
   AFSESSKGLLATSAAKAPGL (SEQ ID NO: 36)
   LRVIAGALEVHAVKPATEEV (SEQ ID NO: 42)
   GELQIVDKIDAAFKIAATAA (SEQ ID NO: 45)
   DAAFKIAATAANAAPTNDKF (SEQ ID NO: 46)
   KFTVFESAFNKALNECTGGA (SEQ ID NO: 48)
   PSLEAAVKQAYAATVAAAPE (SEQ ID NO: 51)
   AYAATVAAAPEVKYAVFEAA (SEQ ID NO: 52)
   PEVKYAVFEAALTKAITAMT (SEQ ID NO: 53)
   AALTKAITAMTQAQKAGKPA (SEQ ID NO: 54)
XXXIX. The peptide according to item XXXVIII wherein said amino acid sequence is derived from one or more of SEQ ID NO: 33, SEQ ID NO: 45, SEQ ID NO: 46, SEQ ID NO: 53 or SEQ ID NO: 54.
XL. An isolated nucleic acid molecule comprising a sequence of nucleotides encoding or complementary to a sequence encoding a peptide according to any one of items I-XXXIX.
XLI. A method for the treatment and/or prophylaxis of a condition in a subject, which condition is characterised by an aberrant, unwanted or otherwise inappropriate immune response to Lol p 1 and/or Lol p 5, said method comprising administering to said subject an effective amount of a peptide according to any one of items I-XXXIX for a time and under conditions sufficient to remove or reduce the presence or function in said subject of T cells directed to said Lol p 1 and/or Lol p 5.
XLII. The method according to item XLI wherein said condition is hypersensitivity to a grass pollen of the subfamily Pooiodeae and even more preferably Rye grass or Timothy grass pollen.
XLIII. Use of a peptide according to any one of items I-XXXIX in the manufacture of a medicament for the treatment of a condition in a mammal which condition is characterised by an aberrant, unwanted or otherwise inappropriate immune response to Lol p 1 and/or Lol p 5.
XLIV. Use according to item XLIII wherein said condition is hypersensitivity to a grass pollen of the subfamily Pooiodeae and even more preferably Rye grass or Timothy grass pollen.
XLV. A pharmaceutical composition comprising a peptide according to any one of items I-XXXIX together with one or more pharmaceutically acceptable carriers and/or diluents.
XLVI. A method of diagnosing or monitoring a condition in a mammal, which condition is characterised by an aberrant, unwanted or inappropriate response to Lol p 1 and/or Lol p 5, said method comprising screening for Lol p 1 and/or Lol p 5 reactive T cells and/or antibodies utilising the peptides according to any one of items I-XXXIX.
XLVII. The method according to item XLVI wherein said condition is hypersensitivity to a grass pollen of the subfamily Pooiodeae and even more preferably Rye grass or Timothy grass pollen.
XLVIII. A diagnostic kit for use in the method of any one of items XLI-XLVII wherein said kit comprises a peptide according to any one of items I-XXXIX.

## Claims

1. An isolated peptide of the formula:
X₁ X₂ X₃
wherein:
X₁ and X₃ may be the same or different and each is an amino acid sequence comprising from 0 to 40 naturally or non-naturally occurring amino acid residues;
X₂ is an amino acid sequence derived from or homologous to, or contiguous with:
a) amino acids 190-245;
b) amino acids 145-173;
c) amino acids 37-83;
d) amino acids 118-137; or
e) amino acids 172-191
inclusive of Lol p 5, provided that X₂ is not the amino acid sequence 100-119 or 190-209; or a functional derivative, homologue, mutant or analogue of said peptide; wherein said peptide molecule is capable of interacting with T cells and modifying T cell function when incubated with cells from subjects having a condition **characterised by** an aberrant, unwanted or otherwise inappropriate immune response to Lol p 5.

2. The peptide according to claim 1, wherein said X₂ amino acid sequence of:
a) is derived from, homologous or contiguous with amino acids 217-236 and/or 226-245, 199-218 or 208-227 inclusive of Lol p 5;
b) is derived from, homologous or contiguous with amino acids 145-164, or 154-173 inclusive of Lol p 5; or
c) is derived from, homologous or contiguous with amino acids 37-56, 46-65, 55-74, or 64-83 inclusive of Lol p 5

3. A peptide according to claim 2 a), wherein said amino acids are 217-236 and/or 226-245 inclusive of Lol p 5.

4. The peptide according to claim 2 b), wherein said amino acids are 145-164 and/or 154-173 inclusive of Lol p 5.

5. The peptide according to claim 2 c), wherein said amino acids are 37-56 inclusive of Lol p 5.

6. The peptide according to claim 1, wherein said amino acid sequence comprises at least 5 amino acids derived from one or more of the following amino acid sequences:
DVNAGFKAAVAAAANAPPAD (SEQ ID NO: 33)
VAAAANAPPADKFKIFEAAF (SEQ ID NO: 34)
ADKFKIFEAAFSESSKGLLA (SEQ ID NO: 35)
AFSESSKGLLATSAAKAPGL (SEQ ID NO: 36)
LRVIAGALEVHAVKPATEEV (SEQ ID NO: 42)
GELQIVDKIDAAFKIAATAA (SEQ ID NO: 45)
DAAFKIAATAANAAPTNDKF (SEQ ID NO: 46)
KFTVFESAFNKALNECTGGA (SEQ ID NO: 48)
PSLEAAVKQAYAATVAAAPE (SEQ ID NO: 51)
AYAATVAAAPEVKYAVFEAA (SEQ ID NO: 52)
PEVKYAVFEAALTKITAMT (SEQ ID NO: 53)
AALTKAITAMTQAQKAGKPA (SEQ ID NO: 54)

7. The peptide according to claim 6 wherein said amino acid sequence is derived from one or more of SEQ ID NO: 33, SEQ ID NO: 45; SEQ ID NO: 46, SEQ ID NO: 53 or SEQ ID NO: 54.

8. An isolated nucleic acid molecule comprising a sequence of nucleotides encoding or complementary to a sequence encoding a peptide according to any one of claims 1 to 7.

9. An isolated peptide as defined in any one of claims 1 to 7 for use in a method for treatment and/or prophylaxis of a condition in a subject, which condition is **characterised by** an aberrant, unwanted or otherwise inappropriate immune response to Lol p 5, said method comprising administering to said subject an effective amount of said peptide for a time and under conditions sufficient to remove or reduce the presence or function in said subject of T cells directed to said Lol p 5.

10. Use of a peptide as defined in any one of claims 1 to 7 in the manufacture of a medicament for the treatment of a condition in a mammal which condition is **characterised by** an aberrant, unwanted or otherwise inappropriate immune response to Lol p 5.

11. The isolated peptide for use according to claim 9, or the use according to claim 10, wherein said condition is hypersensitivity to a grass pollen of the subfamily Pooiodeae and even more preferably Rye grass or Timothy grass pollen.

12. A pharmaceutical composition comprising a peptide according to any one of claims 1 to 7 together with one or more pharmaceutically acceptable carriers and/or diluents.

13. A method of diagnosing or monitoring a condition in a mammal, which condition is **characterised by** an aberrant, unwanted or inappropriate response to Lol p 5, said method comprising screening for Lol p 5 reactive T cells and/or antibodies utilising the peptides as defined in any one of claims 1 to 7.

14. The method according to claim 13, wherein said condition is hypersensitivity to a grass pollen of the subfamily Pooiodeae and even more preferably Rye grass or Timothy grass pollen.

15. A diagnostic kit for use in the method of claim 13 or 14 wherein said kit comprises a peptide as defined in any one of claims 1 to 7.
